(19) Europäisches Patentamt
European Patent Office
Office européen des brevets

(11) Veröffentlichungsnummer: **0 523 533 A1**

(12) **EUROPÄISCHE PATENTANMELDUNG**

(21) Anmeldenummer: **92111597.8**

(22) Anmeldetag: **08.07.92**

(51) Int. Cl.5: **C07D 239/48**, C07D 405/12, C07D 413/12, C07D 401/12, A01N 43/54

(30) Priorität: **16.07.91 DE 4123525**

(43) Veröffentlichungstag der Anmeldung: **20.01.93 Patentblatt 93/03**

(84) Benannte Vertragsstaaten: **AT BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Hamprecht, Gerhard, Dr.**
**Rote-Turm-Strasse 28**
**W-6940 Weinheim(DE)**
Erfinder: **Ditrich, Klaus, Dr.**
**Paray-le-Monial-Strasse 12**
**W-6702 Bad Dürkheim(DE)**
Erfinder: **Westphalen, Karl-Otto, Dr.**
**Mausbergweg 58**
**W-6720 Speyer(DE)**
Erfinder: **Gerber, Matthias, Dr.**
**Ritterstrasse 3**
**W-6704 Mutterstadt(DE)**
Erfinder: **Walter, Helmut, Dr.**
**Grünstadter Strasse 82**
**W-6719 Obrigheim(DE)**

(54) **Substituierte Pyrimidinderivate und ihre Verwendung zur Bekämpfung unerwünschten Pflanzenwuchses.**

(57) Pyrimidinderivate der Formel I

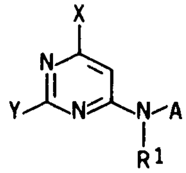

I

in welcher die Substituenten die folgende Bedeutung haben;

| | |
|---|---|
| $R^1$ | Wasserstoff oder Alkyl; |
| A | ein ggf. substituierter bi- oder tricyclischer carbo- oder heterocyclischer Rest, der ein oder mehrere Doppelbindungen enthalten kann; $CR^2R^3R^4$, wobei |
| $R^2$ und $R^3$ | Wasserstoff oder ggf. substituiertes Alkyl und |
| $R^4$ | ggf. substituiertes Naphthyl mit Ausnahme von 1-Naphthyl, ggf. substituiertes Cycloalkyl, ferner falls Y $NH_2$ bedeutet und X nicht für Trifluormethyl steht, Cyclopropyl, ferner ggf. substituiertes Bicycloalkyl oder mono- oder bicyclisches Heterocyclyl mit 1 bis 3 Heteroatomen, ausgewählt aus der Gruppe Sauerstoff, Schwefel und Stickstoff mit Ausnahme von 2-Furyl, 2-Thienyl, 2-Pyrrolyl, 2-N-Methyl-pyrrolyl, 2,3-Pyridyl, 2-Thiolanyl, 2-Perhydro-pyrrolyl und 2-Perhydro-N-methyl-pyrrolyl, falls $R^2$ und $R^3$ jeweils für Wasserstoff stehen, ferner, falls $R^2$ und/oder $R^3$ durch Halogen, Methoxy oder Methylthio substituiert sind, Phenyl, das folgende Gruppen tragen kann: Alkyl, Halogenalkyl, Alkoxy, Halogenalkoxy, Alkylthio, Halogenalkylthio, Halogen, Nitro oder Cyano, bedeuten; |
| X | Halogen, Alkyl, Halogenalkyl, Alkoxy, Halogenalkoxy, Alkylthio, Halogenalkylthio; |

EP 0 523 533 A1

Y Halogen, Alkyl, Halogenalkyl, Alkylthio, ferner, falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch Alkoxy, $C_1$-$C_3$-Alkoxy-$C_2$-$C_3$-alkoxy, $C_1$-$C_3$-Alkylthio-$C_2$-$C_3$-alkoxy,
ferner Y für $NR^5R^6$ steht, wobei

$R^5$ Wasserstoff, Alkyl, Halogenalkyl, Alkoxy, Alkoxyalkyl, Cycloalkyl und

$R^6$ Wasserstoff, außer wenn $R^5$ für Methoxy und $R^4$ für Phenyl stehen, oder Alkyl bedeuten;

sowie deren Additionssalze mit organischen oder anorganischen Säuren.

Die vorliegende Erfindung betrifft neue Pyrimidinderivate der Formel I

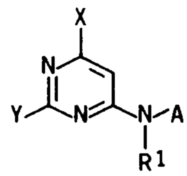

I

in welcher die Substituenten die folgende Bedeutung haben;

- $R^1$ Wasserstoff oder $C_1$-$C_6$-Alkyl;
- A ein bi- oder tricyclischer carbo- oder heterocyclischer Rest, der ein oder mehrere Doppelbindungen enthalten kann und unsubstituiert oder durch folgende Gruppen substituiert ist: $C_1$-$C_3$-Alkyl, $C_1$-$C_3$-Alkoxy oder Halogen; ferner $CR^2R^3R^4$, wobei
- $R^2$ und $R^3$ Wasserstoff, $C_1$-$C_4$-Alkyl oder $C_1$-$C_4$-Alkyl, das ein bis drei der folgenden Gruppen trägt: $C_1$-$C_4$-Alkoxy, $C_1$-$C_4$-Alkylthio oder Halogen und
- $R^4$ unsubstituiertes oder substituiertes Naphthyl mit Ausnahme von 1-Naphthyl;
  unsubstituiertes oder substituiertes $C_4$-$C_8$-Cycloalkyl, ferner Cyclopropyl, falls Y $NH_2$ bedeutet und X nicht für Trifluormethyl steht;
  unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff mit Ausnahme von 2-Furyl, 2-Thienyl, 2-Pyrrolyl, 2-N-Methyl-pyrrolyl, 2,3-Pyridyl, 2-Thiolanyl, 2-Perhydro-pyrrolyl und 2-Perhydro-N-methylpyrrolyl, falls $R^2$ und $R^3$ jeweils für Wasserstoff stehen und Y keine $NH(C_1$-$C_4$-Alkoxy)-Gruppe bedeutet, ferner falls Y eine $NH(C_1$-$C_4$-Alkoxy)-Gruppe bedeutet, unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff;
  Phenyl, falls $R^2$ und/oder $R^3$ durch Halogen, Methoxy oder Methylthio substituiert sind, oder falls Y $NH_2$ und gleichzeitig X $C_1$-$C_3$-Halogenalkyl oder $C_1$-$C_3$-Halogenalkoxy bedeuten oder falls Y Mono- oder Di-$C_1$-$C_6$-alkylamino und gleichzeitig X $C_1$-$C_3$-Halogenalkoxy bedeuten, wobei der Phenylrest folgende Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro oder Cyano;
  $C_2$-$C_6$-Alkyl, $C_2$-$C_6$-Alkenyl, $C_2$-$C_6$-Alkinyl, falls Y für $NH(C_1$-$C_4$-Alkoxy) und gleichzeitig X für Chlor oder $C_1$-$C_3$-Halogenalkyl steht;
- X Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Halogenalkylthio;
- Y Halogen, $C_1$-$C_3$-Alkyl, $C_1$-$C_3$-Halogenalkyl, $C_1$-$C_3$-Alkylthio, ferner, falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch $C_1$-$C_4$-Alkoxy, $C_1$-$C_3$-Alkoxy-$C_2$-$C_3$-alkoxy, $C_1$-$C_3$-Alkylthio-$C_2$-$C_3$-alkoxy,
  ferner Y für $NR^5R^6$ steht, wobei
- $R^5$ Wasserstoff, $C_1$-$C_6$-Alkyl, $C_1$-$C_3$-Halogenalkyl, $C_1$-$C_4$-Alkoxy, $C_1$-$C_3$-Alkoxy-$C_1$-$C_3$-alkyl, $C_3$-$C_6$-Cycloalkyl und
- $R^6$ Wasserstoff, außer wenn $R^5$ für eine Methoxygruppe und $R^4$ für Phenyl stehen, oder $C_1$-$C_4$-Alkyl bedeuten,

sowie deren Additionssalze mit organischen oder anorganischen Säuren.

Ferner betrifft die Erfindung Verfahren zur Herstellung der Pyrimidinderivate und ihre Verwendung zur Bekämpfung unerwünschten Pflanzenwuchses.

Eine Reihe von Pyrimidinderivaten mit herbizider Wirkung sind bekannt. In DE-A 20 65 629 und DE-A 20 06 145 werden herbizide 2-Amino-6-chlor-pyrimidinderivate mit einer Alkyl-, Allyl- oder Cyclohexylaminogruppe in 4-Position beschrieben.

Weitere herbizide Derivate werden in DE-A 26 30 140, DE-A 36 01 800, EP-A 254 183, EP-A 332 963 und DE-A 37 17 480 und den japanischen Offenlegungsschriften JO 1-250-363 (27.12.88) und J6 3 313-777 (17.06.87) offenbart. Diese Verbindungen lassen jedoch wegen der unbefriedigenden Selektivität gegenüber Schadpflanzen zu wünschen übrig.

Der Erfindung lagen daher neue Verbindungen aus der Klasse substituierter Pyrimidine mit verbesserten herbiziden Eigenschaften als Aufgabe zugrunde. Entsprechend dieser Aufgabe wurden die eingangs definierten Pyrimidine gefunden.

Ferner wurde gefunden, daß die Verbindungen der Formel I sowie deren Salze eine gute Selektivität gegen Schadpflanzen in Kulturen wie Getreide und Mais haben.

Unter Salzen sind Additionsalze folgender anorganischer und organischer Säuren zu verstehen: Halogenwasserstoffsäuren wie Chlorwasserstoff- und Bromwasserstoffsäure, Phosphorsäure, Schwefelsäure, Fluorborsäure ($HBF_4$), Perchlorsäure, Alkylschwefelsäuren wie Methyl- oder Ethylschwefelsäure, Naphthoesäuren, Benzoesäure, Halogenbenzoesäuren, Essigsäure, Halogenessigsäuren wie Trichloressigsäure, Aminoessigsäure, Propionsäure, Halogenpropionsäuren, Buttersäure, Milchsäure, Stearinsäure, aliphatische Dicarbonsäuren wie Oxalsäure, Weinsäure, Maleinsäure; aromatische Sulfonsäuren wie p-Toluolsulfon säure etc.

Bei optisch aktiven Verbindungen der Formel I umnfaßt die Erfindung sowohl die Racemate als auch die möglichen Enantiomere.

Als herbizide Wirkstoffe bevorzugte Pyrimidinderivate sind solche der Formel I in der $R^1$ Wasserstoff bedeutet.

Weiterhin sind Pyrimidinderivate der Formel I bevorzugt, bei denen

- $R^1$ für Wasserstoff,
- X für Halogen,
- Y für $NHR^5$, wobei
- $R^5$ Wasserstoff, $C_1$-$C_6$-Alkyl, $C_1$-$C_3$-Alkoxy-$C_1$-$C_3$-alkyl, $C_1$-$C_4$-Alkoxy außer Methoxy, falls $R^4$ Phenyl bedeutet, darstellt;
- A für einen bi- oder tricyclischen, carb- oder heterocyclischen Rest, der ein oder mehrere Doppelbindungen enthalten kann und unsubstituiert oder durch Methyl, Methoxy oder Halogen substituiert ist,

stehen, ferner A für $CHR^3R^4$ steht, wobei

- $R^3$ Wasserstoff, $C_1$-$C_4$-Alkyl oder Trifluormethyl und
- $R^4$ die eingangs genannte Bedeutung hat.

Besonders bevorzugt sind Pyrimidinderivate der Formel I, in der die Substituenten folgende Bedeutung haben:

- $R^1$ Wasserstoff,;
- X Chlor;
- Y $NH_2$;
- A eine Gruppe $CHR^3R^4$;
- $R^3$ Wasserstoff, $C_1$-$C_4$-Alkyl;
- $R^4$ 2-Naphthyl, $C_3$-$C_8$-Cycloalkyl, Bicyclo[2.2.1]hept-2-yl, 2-Benzofuryl, 3-Thienyl, 2-Tetrahydrofuryl, 2-Indanyl, 2-Tetrahydroindanyl, 3-Benzopyranyl;

ferner

- $R^1$ Wasserstoff,;
- X Chlor;
- Y $NH(C_1$-$C_4)$-Alkoxy;
- A eine Gruppe $CHR^3R^4$;
- $R^3$ Wasserstoff, $C_1$-$C_4$-Alkyl;
- $R^4$ $_1$-Naphthyl, $C_3$-$C_8$-Cycloalkyl, Phenyl, 3-Chlorphenyl, 2-Furyl, 3-(1-Methylethyl)isoxazol-5-yl, 3-Thienyl, 3-Tetrahydrofuryl, 2-Tetrahydroindanyl;

ferner

- $R^1$ Wasserstoff,;
- X Trifluormethoxy;
- Y $NH_2$;
- A eine Gruppe $CHR^3$, $R^4$;
- $R^3$ Wasserstoff, $C_1$-$C_4$-Alkyl;
- $R^4$ 1-Naphthyl, $C_3$-$C_8$-Cycloalkyl, Phenyl, 3-Thienyl, 2-Benzofuranyl;

ferner

- $R^1$ Wasserstoff,;
- X Trifluormethyl;
- Y $NH_2$;
- A eine Gruppe $CHR^3$, $R^4$;
- $R^3$ Wasserstoff, $C_1$-$C_4$-Alkyl;
- $R^4$ 1-Naphthyl, $C_3$-$C_8$-Cycloalkyl, Phenyl, 3-Thienyl, 2-Tetrahydrofuryl, 1-Indanyl.

Weiterhin betrifft die Erfindung Verfahren zur Herstellung dieser Verbindungen, sowie herbizide Mittel, welche mindestens eine Verbindung I enthalten, in der die Substituenten die vorstehend gegebene Bedeutung haben.

Die erfindungsgemäßen Pyrimidinderivate sind auf verschiedenen Wegen herstellbar; man erhält sie beispielsweise nach den folgenden Verfahren:

1. Durch Umsetzung von Pyrimidinen der Formel II

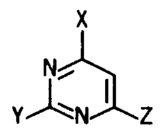

II

worin X und Y die vorgenannte Bedeutung besitzen und Z für eine nucleophil austauschbare Gruppe, wie Halogen, vorzugsweise Fluor, Chlor oder Brom steht, mit einem Amin der Formel III

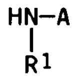

III

in der $R^1$ und A die vorgenannte Bedeutung besitzen.

Die Reaktion wird zweckmäßigerweise so durchgeführt, daß man die Pyrimidine der Formel II in einem inerten organischen Lösungsmittel vorlegt, eine Mischung des Amins III mit einer organischen Base zugibt und dann bei erhöhter Temperatur die Umsetzung zu Ende führt.

Das Gemisch kann wie üblich aufgearbeitet werden, beispielsweise durch Extraktion mit Wasser zur Entfernung des Basenhydrochlorids, Absaugen oder Extraktion des Produktes mit einem organischen Lösungsmittel und Einengen des organischen Lösungsmittels.

Zweckmäßigerweise vewendet man für diese Umsetzungen Lösungsmittel wie Halogenkohlenwasserstoffe, z. B. Tetrachlorethan, Methylenchlorid, Chloroform, Dichlorethan, Chorbenzol und 1,2-Dichlorbenzol; Ether, z. B. Diethylether, Methyl-tert.-butylether, Dimethoxiethan, Diethylenglykoldiethylether, Tetrahydrofuran und Dioxan; dipolare aprotische Lösungsmittel, z. B. Acetonitril, Dimethylformamid, Dimethylacetamid, Dimethylsulfoxid, N-Methylpyrrolidon, 1,3-Dimethyltetrahydro-2(1H)-pyrimidinon, 1,3-Dimethylimidazolin-2-on; Aromaten, z. B. Benzol, Toluol, Xylol, Pyridin und Chinolin; Ketone, z. B. Aceton, Methylethylketon; Alkohole, z. B. Ethanol, Propanol und Isopropanol oder entsprechende Gemische.

Die Umsetzung kann bei Temperaturen von $50\,^{\circ}C$ bis zur Rückflußtemperatur des jeweiligen Lösungsmittels bzw. -gemisches, vorzugsweise bei 70 bis $150\,^{\circ}C$, durchgeführt werden.

Die molaren Verhältnisse, in denen die benötigten Ausgangsverbindungen miteinander umgesetzt werden, betragen 0,9:1 bis 1,3:1 für das Verhältnis von Amin III, bzw. organischen Base zu Pyrimidin II. Man kann jedoch das Amin III auch in einem Überschuß von etwa 2 mol pro Mol Pyrimidin einsetzen.

Die Konzentration der Edukte im Lösungsmittel(gemisch) beträgt im allgemeinen 0,1 bis 5 mol/l, bevorzugt 0,2 bis 2 mol/l.

2. Ein weiteres Verfahren zur Herstellung der Verbindungen der Formel I beruht auf der Umsetzung eines Pyrimidins der Formel IV in der X die vorgenannte Bedeutung hat und Z für Halogen steht, zunächst mit einem ersten Nucleophil der Formel V, in der Y für einen vorgenannten Sauerstoff- oder Stickstoff-gebundenen Rest steht, zu den Verbindungen der Formel II, die dann mit dem Amin der Formel III zu den Endstoffen I umgesetzt werden.

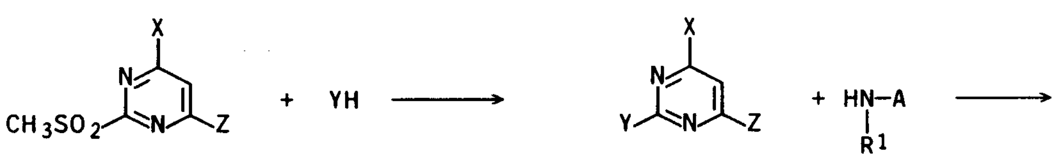

IV V II III

Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetra-hydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon, Alkohole, z.B. Ethanol, Propanol und Isopropanol oder entsprechende Gemische.

Mitunter ist es nützlich, den ersten Reaktionsschritt in Gegenwart eines Säureakzeptors durchzuführen.

Als Säureakzeptoren setzt man vorzugsweise aromatische Stickstoffbasen, wie Pyridin, 4-Dimethylaminopridin und Chinolin; tertiäre aliphatische Amine, wie Triethylamin, N-Ethyl-diisopropylamin und N-Methylmorpholin; bi- und tricyclische Amine, wie Diazabicyclooctan (DABCO) und Diazabicycloundecan (DBU); oder auch Carbonate bzw. Hydrogencarbonate von Alkali- oder Erdalkalimetallen, wie z.B. Natriumcarbonat, Kaliumcarbonat, Natriumhydrogencarbonat und Kaliumhydrogencarbonat ein. Mitunter ist es auch nützlich Kombinationen der oben angeführten Basen zu verwenden.

Das molare Verhältnis, in dem Nucleophil V und Säureakzeptor eingesetzt werden, beträgt im allgemeinen 1:0,5 bis 1:5.

Die Umsetzung kann in der ersten Stufe bei Temperaturen von -80 °C bis 150 °C, vorzugsweise von -80 bis 60 °C, durchgeführt werden und folgt dann in der 2. Stufe dem Verfahren 1.

Die molaren Verhältnisse, in denen die benötigten Ausgangsverbindungen miteinander umgesetzt werden, betragen im allgemeinen 0,9:1 bis 1,2:1 für das Verhältnis von Nucleophil V zu Pyrimidin IV.

Die Konzentration der Edukte im Lösungsmittel(gemisch) beträgt im allgemeinen 0,1 bis 5 mol/l, bevorzugt 0,2 bis 2 mol/l.

Nach erfolgter Umsetzung wird das Reaktionsgemisch der ersten Stufe mit Wasser gewaschen, getrocknet und eingeengt.

3. Ein weiteres Verfahren zur Herstellung von Verbindungen der Formel Ia,

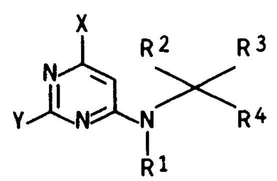

Ia,

$R^4$ = Heterocyclus

in der die Substituenten X, Y, $R^1$, $R^2$ und $R^3$ die vorstehende Bedeutung haben und $R^4$ für einen fünfgliedrigen heterocyclischen Rest steht, besteht darin, daß man ein Alkin der Formel VI

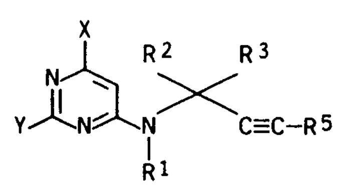

VI

(herstellbar nach Verfahren 1 oder 2), in der die Substituenten X, Y, $R^1$, $R^2$ und $R^3$ die vorstehende Bedeutung haben und $R^5$ für Wasserstoff oder $C_1$-$C_4$-Alkyl steht, mit einem 1,3-Dipol VII

$$\overset{\oplus}{a}=b-\overset{\ominus}{c} \qquad VII$$

im Sinne einer Cycloadditon umsetzt. Die 1,3-Dipole VII können nach bekannten Verfahren (siehe A. Padwa, "1,3-Dipolar Cycloadditon Chemistry"; Wiley, New York 1984) erzeugt und umgesetzt werden.

Beispielhaft sei hier das Verfahren zur Herstellung von Verbindungen der Formel Ib

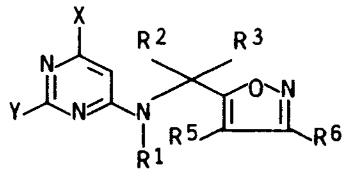

Ib ,

in der die Substituenten X, Y, $R^1$, $R^2$, $R^3$ und $R^5$ die vorstehende Bedeutung haben und $R^6$ für Wasserstoff, Halogen, $C_1$-$C_4$-Alkyl oder ggf. substituiertes Phenyl steht, angeführt.

Man geht dabei so vor, daß man ein Alkin der Formel VI in der die Substituenten die voranstehende Bedeutung haben, mit einem Hydroxamsäurechlorid der Formel VIII, in der $R^6$ die obenstehende Bedeutung hat in Gegenwart einer Stickstoffbase umsetzt.

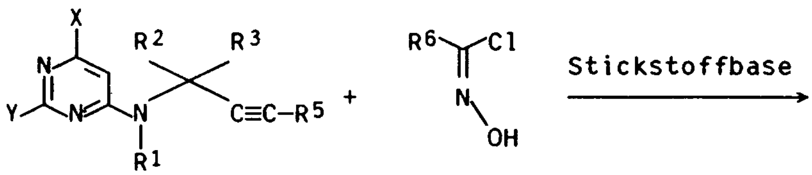


Ib

VI VIII

Dabei geht man zweckmäßigerweise so vor, daß man das Alkin VI in einem inerten organischen Lösungsmittel vorlegt, etwa molare Mengen des Hydroxamsäurechlorids VIII zugibt und anschließend eine etwa doppeltmolare Menge einer Base zutropft. Das Gemisch kann wie üblich aufgearbeitet werden, z. B. durch Hydrolyse mit Wasser und Absaugen oder Extraktion des Produktes mit einem organischen Lösungsmittel.

Als Lösungsmittel für diese Umsetzungen verwendet man zweckmäßigerweise Halogenkohlenwasserstoffe wie Dichlorethan, Chlorbenzol, 1,2-Dichlorbenzol, Tetrachlorethan, Dichlormethan und Chloroform; Ether wie Diethylether, Methyl-tert.-butylether, Dimetoxyethan, Diethylenglykoldimethylether, Tetrahydrofuran und Dioxan oder Aromaten wie Benzol, Toluol und Xylol oder Gemische dieser Lösungsmittel.

Die Umsetzungen können bei Temperaturen von -10 °C bis 50 °C, vorzugsweise bei 0 bis 30 °C, durchgeführt werden.

Als Basen kommen vorzugsweise Stickstoffbasen wie 2-, 3-, 4-Picolin, N,N-Dimethylanilin, N,N-Dimethylcyclohexylamin, Triethylamin (TEA), Pyridin und 1,8-Diazabicyclo(5.4.0)undec-7-en (DBU) in Betracht.

Die Reaktionspartner werden insbesondere im Molverhältnis 1:1 eingesetzt, die Stickstoffbase wird in der doppelt bis dreifach molaren Menge zugesetzt.

Die Konzentration der Edukte im Lösungsmittelgemisch beträgt im allgemeinen 0,1 bis 5 mol/l, bevorzugt 0,2 bis 2 mol/l.

Im Hinblick auf die bestimmungsgemäße Verwendung der Verbindungen I kommen als Substituenten bespielsweise folgende Reste in Betracht:

$R^1$ Wasserstoff, $C_1$-$C_6$-Alkyl wie Methyl, Ethyl, Propyl, 2-Methylethyl, Butyl, 1-Methylpropyl, 2-Methylpropyl, 1,1-Dimethylethyl, Pentyl, 1-Methylbutyl, 2-Methylbutyl, 3-Methyl-butyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,2,2-Trimethylpropyl, 1,2,3-Trimethypropyl, 1-Ethyl-1-methylpropyl und 1-Ethyl-2-methylpropyl, insbesondere Methyl, Ethyl, Propyl, 1-Methylethyl, und 1,1-Dimethylethyl;

A ein bi- oder tricyclischer carbo- oder heterocyclischer Rest, der gesättigt ist, oder ein oder mehrere Doppelbindungen enthält, z.B. aromatisch ist und ausgewählt ist aus der Gruppe: 2-, 3-, 4-, 5-, 6-, 7-Benzofuryl, 1-, 3-, 4-, 5-, 6-, 7-Isobenz-furyl, 2-, 3-, 4-, 5-, 6-, 7-Benzo-[b]thienyl, 1-, 3-, 4-, 5-, 6-, 7-Benzo-[c]thienyl, 2-, 3-, 4-, 5-, 6-, 7-Indolyl, 1-, 3-, 4-, 5-, 6-, 7-Isoindolyl, 3-, 4-, 5-, 6-, 7-Benz[c] oder -[b]isoxazolyl, 3-, 4-, 5-, 6-, 7-Benz[c] oder -[b]-isothiazolyl, 3-, 4-, 5-, 6-, 7-Benz[b] oder [c]pyrazolyl, 2-, 4-, 5-, 6-, 7-Benzoxazolyl, 2-, 4-, 5-, 6-, 7-Benzthiazolyl, 2-, 4-, 5-, 6-Benzimidazolyl, 2-, 3-, 4-, 5-, 6-, 7-[2,3 Dihydrobenzofuryl], 1-, 3-, 4-, 5-, 6-, 7-[1,3-Dihydro-isobenzofuryl], 2-, 3-, 4-, 5-, 6-, 7-[2,3-Dihydrobenzo[b]thienyl], 1-, 3-, 4-, 5-, 6-, 7-[1,3-Dihydrobenzo[c]thienyl], 1-, 3-, 4-, 5-, 6-, 7-[1,3-Dihydro-benzo[c]thienyl], 1-, 3-, 4-, 5-, 6-, 7-[1,3-Dihydro-isoindol], 2-, 3-, 4-, 5-, 6-, 7-[2,3-Dihydro-indol], 2-, 3-, 4-, 5-, 6-, 7-, 8-Chromenyl, 1-, 3-, 4-, 5-, 6-, 7-, 8-Isochromenyl, 2-, 3-, 4-, 5-, 6-, 7-, 8-Chinolyl, 1-, 3-, 4-, 5-, 6-, 7-, 8-Isochinolyl,

2-, 3-, 4-, 5-, 6-, 7-, 8-Chromanyl, 1-, 3-, 4-, 5-, 6-, 7-, 8-, Isochromanyl, 3-, 4-, 5-, 6-, 7-, 8-Cinnolinyl, 1-, 4-, 5-, 6-, 7-, 8-Phthalazinyl, 2-, 3-, 5-, 6-, 7-, 8-Chinoxalinyl, 2-, 4-, 5-, 6-, 7-, 8-Chinazolinyl, 3-, 5-, 6-, 7-, 8-Benzo-1,2,4-triazinyl, 2-, 3-, 4-, 5-, 6-, 7-Benzopyranyl, 2-, 3-, 4-, 5-, 6-, 7-Naphthyridinyl, 2-, 3-, 4-, 6-, 7-, 8-Pyrido[3,2-b]pyridinyl, 2-, 3-, 4-, 5-, 7-, 8-Pyrido[4,3-b]pyridinyl, 2-, 3-, 4-, 5-, 6-,8-Pyrido[3,4-b]pyridinyl, 2-, 4-, 5-, 6-, 7-, 8-[1,3,2-Benzoxazinyl], 2-, 3-, 5-, 6-, 7-, 8-[1,4,2-Benzoxazinyl], 1-, 4-, 5-, 6-, 7-, 8-[2,3,1-Benzoxazinyl], 2-, 4-, 5-, 6-, 7-, 8-[3,1,4-Benzoxazinyl], 3-, 4-, 5-, 6-, 7-, 8-[1,2-Benzisoxazinyl, 2-, 3-, 5-, 6-, 7-, 8-[1,4-Benzisoxazinyl], 2-, 6-, 8-Purinyl, 1-, 2-, 3-, 4-, 5-, 6-, 7-Indanyl, 1-, 2-, 3-, 4-, 5-, 6-, 7-Indenyl, 1-, 2-, 3-, 4-, 5-, 6-, 7-, 8-Tetralinyl, 1-, 2-, 3-, 4-, 5-, 6-, 7-, 8-Dekalinyl, 3-, 4-, 5-, 7-, 8-, 9-, 10-Tricyclo-[5.2.1.$0^{2,6}$]decanyl, 3-, 4-, 5-, 7-, 8-, 9-, 10-Tricyclo-[5.2.1.$0^{2,6}$]-dec-$2^6$-enyl, 1-, 2-Norbornyl, 2-Bornyl, 2-, 3-Norpinyl, 1-, 2-, 3-, 4-, 5-, 6-, 7-, 8-, 9-Fluorenyl, 1-, 2-, 3-, 4-, 5-, 6-, 7-, 8-Naphthyl;

wobei die genannten Reste eine bis drei der folgenden Gruppen tragen können:

$C_1$-$C_3$-Alkyl wie Methyl, Ethyl, Propyl und 1-Methylethyl; $C_1$-$C_3$-Alkoxy wie Methoxy, Ethoxy, Propoxy und 1-Methyl-ethoxy; Halogen wie Fluor, Chlor, Brom, Jod, insbesondere Fluor und Chlor;

$R^2$ und $R^3$ Wasserstoff, $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl, Ethyl, Propyl und 1-Methylethyl, welches eine bis drei der folgenden Gruppen tragen kann:

$C_1$-$C_4$-Alkoxy wie Methoxy, Ethoxy, Propoxy, 1-Methylethoxy, Butoxy, 1-Methylpropyloxy, 2-Methylpropyloxy und 1,1-Dimethylethoxy, insbesondere Methoxy, Ethoxy, Propoxy und 1-Methylethoxy; C1-C4-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio, Ethylthio, Propylthio und 1-Methylethylthio;

Halogen, wie Fluor, Chlor, Brom, Jod, insbesondere Fluor und Chlor;

$R^4$ substituiertes 1-Naphthyl, ferner 2-Naphthyl, ferner falls $Y = NH_2$ bedeutet und X nicht für Trifluormethyl steht, Cyclopropyl;

$C_4$-$C_8$-Cycloalkyl wie Cyclobutyl, Cyclopentyl, Cyclohexyl, Cycloheptyl und Cyclooctyl, insbesondere Cyclopentyl und Cyclohexyl,

mono- oder bicyclisches Heterocyclyl mit 1 bis 3 Heteroatomen ausgewählt aus der Gruppe Sauerstoff, Schwefel und Stickstoff, wie 2-Furyl, 2-Thienyl, 2-Pyrrolyl, 2-N-Methylpyrrolyl, 2-, 3-Pyridyl, 2-Thiolanyl, 2-Perhydro-pyrrolyl, 2-Perhydro-N-methyl-pyrrolyl, wobei $R^2$ und $R^3$ nicht zusammen für Wasserstoff stehen,

ferner 2,3-Tetrahydrofuryl, 3-Furyl, 3-Perhydro-pyrrolyl, 3-Pyrrolyl, 3-Thiolanyl, 3-Thienyl, 2-, 3-, 4-Pyranyl, 2-, 3-,4-Dihydropyranyl, 2-, 3-, 4-Tetrahydropyranyl, 2-, 3-, 4-Thiopyranyl, 2-, 3-, 4-Tetrahydro-thiopyranyl, 4-Pyridyl, 3-, 4-, 5-Isoxazolyl, 3-, 4-, 5-Isothiazolyl, 2-, 4-, 5-Oxazolyl, 2-, 4-, 5-Thiazolyl, 2-, 4-, 5-Imidazolyl, 3-, 4-Pyrazolyl, 2-Pyrazinyl, 2-, 4-, 5-Pyrimidinyl, 2-, 3-Pyridazinyl, 2-, 3- Dioxolanyl, 4-, 5-[1,2,3-Thiadiazolyl], 4-, 5-[1,2,3-Oxadiazolyl], 3-, 5-[1,2,4-Oxadiazolyl], 2-, 5-[1,3,4-Oxadiazolyl], 2-, 4-, 5-, 6-[1,3-Dioxinyl], 2-, 4-, 5-, 6-[1,3-Dioxanyl], 2-, 3-, 5-, 6-[1,4-Dioxanyl], 2-, 4-, 6-[1,3,5-Triazinyl], 3-, 5-, 6-[1,2,4-Triazinyl], 3-, 4-, 5-, 6-[1,2,4-Oxazinyl], 2-, 4-, 5-, 6-[1,3,2-Oxazinyl], 2-, 4-, 5-, 6-[1,3,6-Oxazinyl], 3-, 4-, 5-, 6-[1,2,6-Oxazinyl], 2-, 3-, 5-, 6-[1,4-Oxazinyl],

sowie die bei A genannten Heterocyclen und Bialicyclen, welche eine bis drei der folgenden Gruppen tragen können:

$C_1$-$C_3$-Alkyl wie Methyl, Ethyl, Propyl und 1-Methylethyl; $C_1$-$C_3$-Alkoxy wie Methoxy, Ethoxy, Propoxy und 1-Methylethoxy; Halogen wie Fluor, Chlor, Brom, Jod insbesondere Fluor und Chlor, Phenyl, das folgende Gruppen tragen kann: Methyl, Ethyl, Trifluormethyl, Methoxy, Ethoxy, Difluormethoxy, Trifluormethoxy, Chlordifluormethoxy, Methylthio, Difluormethylthio, Trifluormethylthio, Chlordifluormethylthio, Halogen, Nitro oder Cyano,

X Halogen wie vorstehend genannt, insbesondere Chlor und Brom; $C_1$-$C_3$-Alkyl wie vorstehend genannt, insbesondere Methyl und Ethyl; $C_1$-$C_3$-Halogenalkyl wie Trifluormethyl, Chlordifluormethyl, Tetrafluorethyl, Pentafluorethyl, insbesondere Trifluor- und Chlordifluormethyl;

$C_1$-$C_3$-Alkoxy wie vorstehend genannt, insbesondere Methoxy und Ethoxy;

$C_1$-$C_3$-Halogenalkoxy wie Difluormethoxy, Trifluormethoxy, Chlordifluormethoxy, Tetrafluorethoxy, Pentafluorethoxy insbesondere Difluormethoxy, Trifluormethoxy und Chlordifluormethoxy; $C_1$-$C_3$-Alkylthio wie vorstehend genannt, insbesondere Methylthio und Ethylthio; $C_1$-$C_3$-Halogenalkylthio wie Difluormethylthio, Trifluormethylthio, Chordifluormethylthio, Tetrafluorethylthio, Pentafluorethylthio, insbesondere Difluormethylthio, Trifluormethylthio und Chlordifluormethylthio;

Y Halogen wie vorstehend genannt, insbesondere Chlor und Brom; $C_1$-$C_3$-Alkyl wie vorstehend genannt, insbesondere Methyl und Ethyl; $C_1$-$C_4$-Alkoxy wie vorstehend genannt, insbesondere Methoxy, Ethoxy und Propoxy; $C_1$-$C_3$-Alkoxy-$C_2$-$C_3$-alkoxy wie Methoxyethoxy, Ethoxyethoxy, Methoxypropoxy, Ethoxyethoxy, insbesondere Methoxyethoxy und Ethoxyethoxy; $C_1$-$C_3$-Alkylthio wie vorstehend genannt, insbesondere Methylthio und Ethylthio; $C_1$-$C_3$-Halogenalkyl wie vorstehend genannt, insbesondere Trifluormethyl, $C_1$-$C_3$-Alkylthio-$C_2$-$C_3$-alkoxy wie Methylthioethoxy, Ethylthio-ethoxy, Methylthiopropoxy, insbesondere Methylthioethoxy und Ethylthioethoxy,

$R^5$ Wasserstoff, $C_1$-$C_6$-Alkyl wie bei $R^1$ genannt, insbesondere Methyl, Ethyl, Propyl und 1-Methylethyl, $C_1$-$C_4$-Alkoxy wie vorgenannt, insbesondere Methoxy, Ethoxy und Propoxy, $C_1$-$C_3$-Alkoxy-$C_2$-$C_3$-alkyl wie Methoxyethyl, Methoxypropyl, Ethoxyethyl, Ethoxypropyl, insbesondere Methoxyethyl; $C_3$-$C_8$-Cycloalkyl wie bei $R^4$ genannt, insbesondere Cyclopropyl, Cyclopentyl und Cyclohexyl;

$R^6$ Wasserstoff, $C_1$-$C_4$-Alkyl wie vorstehend genannt, insbesondere Methyl und Ethyl.

Die Pyrimidinderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,02 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 %

bis 100 % (nach NMR-Spektrum) eingesetzt.

Die erfindinngsgemäßen Verbindungen I können beispielsweise wie folgt formuliert werden:

I. Man vermischt 90 Gewichtsteile der Verbindung Nr. 19 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 10 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 20 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 13 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 19 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 10 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 20 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 13 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel bzw. der Wirkstoffe kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 5 kg/ha, vorzugsweise 0,01 bis 2,0 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindinngsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |

| Botanischer Name | Deutscher Name |
|---|---|
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus dulcis | Mandelbaum |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Pyrimidinderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazin, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Sulfonylharnstoffderivate, Cyclohexan-1,3-dionderivate, (Hetero)-aryloxy-phenoxy-propionsäuren, deren Salze, Ester und Amide und andere in Betracht.

Außerdem kann es von Nutzen sein, die Pyrimidinderivate der Formel I bzw. sie enthaltende herbide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Beispiele für herbizid wirksame Verbindungen mit der allgemeinen Struktur I sind nachstehend aufgeführt.

(bzw. $NR^5R^6$) 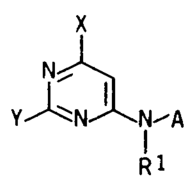 (bzw. $CR^2R^3R^4$)

wobei

- X für einen Rest aus der Gruppe Q1 bis Q30
- Y für einen Rest aus der Gruppe L1 bis L32
- $R^1$ für einen Rest aus der Gruppe M1 bis M34

A für einen Rest aus der Gruppe N1 bis N317
$R^2$ für einen Rest aus der Gruppe O1 bis O33
$R^3$ für einen Rest aus der Gruppe P1 bis P33
$R^4$ für einen Rest aus der Gruppe R1 bis R150
$R^5$ für einen Rest aus der Gruppe S1 bis S53
$R^6$ für einen Rest aus der Gruppe T1 bis T9

stehen, die Reste Q, L, M, N, O, P, R, S und T beliebig kombiniert werden können und im einzelnen die folgende Bedeutung haben.

| Verb. Nr. | X |
|---|---|
| Q 1 | Cl |
| Q 2 | Br |
| Q 3 | J |
| Q 4 | F |
| Q 5 | $CH_3$ |
| Q 6 | $C_2H_5$ |
| Q 7 | n-$C_3H_7$ |
| Q 8 | i-$C_3H_7$ |
| Q 9 | $CF_3$ |
| Q 10 | $CF_2Cl$ |
| Q 11 | $HCF_2CF_2$ |
| Q 12 | $CF_2CF_3$ |
| Q 13 | $CH_3O$ |
| Q 14 | $C_2H_5O$ |
| Q 15 | n-$C_3H_7O$ |
| Q 16 | i-$C_3H_7O$ |
| Q 17 | $HCF_2O$ |
| Q 18 | $CF_3O$ |
| Q 19 | $CF_2ClO$ |
| Q 20 | $HCF_2CF_2O$ |
| Q 21 | $CF_3CF_2O$ |
| Q 22 | $CH_3S$ |
| Q 23 | $C_2H_5S$ |
| Q 24 | n-$C_3H_7S$ |
| Q 25 | i-$C_3H_7S$ |
| Q 26 | $HCF_2S$ |
| Q 27 | $CF_3S$ |
| Q 28 | $CF_2ClS$ |
| Q 29 | $HCF_2CF_2S$ |
| Q 30 | $CF_3CF_2S$ |

| Verb. Nr. | Y |
|---|---|
| L 1 | Cl |
| L 2 | Br |
| L 3 | J |
| L 4 | F |
| L 5 | $CH_3$ |
| L 6 | $C_2H_5$ |
| L 7 | n-$C_3H_7$ |
| L 8 | i-$C_3H_7$ |
| L 9 | $CH_3O$ |
| L 10 | $C_2H_5O$ |
| L 11 | n-$C_3H_7O$ |
| L 12 | i-$C_3H_7O$ |
| L 13 | n-$C_4H_9O$ |
| L 14 | sec-$C_4H_9O$ |
| L 15 | tert.-$C_4H_9O$ |
| L 16 | i-$C_4H_9O$ |
| L 17 | $CH_3O[CH_2]_2O$ |
| L 18 | $CH_3O$-$[CH_2]_3O$ |
| L 19 | $C_2H_5O[CH_2]_2O$ |
| L 20 | n-$C_3H_7O[CH_2]_2O$ |
| L 21 | i-$C_3H_7O[CH_2]_2O$ |
| L 22 | $CH_3S$ |
| L 23 | $C_2H_5S$ |
| L 24 | n-$C_3H_7S$ |
| L 25 | i-$C_3H_7S$ |
| L 26 | $CF_3$ |
| L 27 | $CF_2Cl$ |
| L 28 | $HCF_2CF_2$ |
| L 29 | $CF_3CF_2$ |
| L 30 | $CF_3[CF_2]_2$ |
| L 31 | $CH_3S[CH_2]_2O$ |
| L 32 | $CH_3S[CH_2]_3O$ |

| Verb. Nr. | R1 |
|---|---|
| M 1 | H |
| M 2 | $CH_3$ |
| M 3 | $C_2H_5$ |
| M 4 | n-$C_3H_7$ |
| M 5 | i-$C_3H_7$ |
| M 6 | n-$C_4H_9$ |
| M 7 | i-$C_4H_9$ |
| M 8 | sek.-$C_4H_9$ |
| M 9 | tert.-$C_4H_9$ |
| M 10 | n-$C_5H_{11}$ |
| M 11 | $CH[CH_3]C_3H_7$ |
| M 12 | $CH_2CH[CH_3]C_2H_5$ |
| M 13 | $[CH_2]_2CH[CH_3]CH_3$ |
| M 14 | $C[CH_3]_2C_2H_5$ |
| M 15 | $CH[CH_3]CH[CH_3]CH_3$ |
| M 16 | $CH_2$-$C[CH_3]_2CH_3$ |
| M 17 | $CH[C_2H_5]C_2H_5$ |
| M 18 | n-$C_6H_{13}$ |
| M 19 | $CH[CH_3]C_4H_9$ |
| M 20 | $CH_2CH[CH_3]C_3H_7$ |
| M 21 | $[CH_2]_2CH[CH_3]C_2H_5$ |
| M 22 | $[CH_2]_3CH[CH_3]CH_3$ |
| M 23 | $C[CH_3]_2C_3H_7$ |
| M 24 | $CH[CH_3]CH[CH_3]C_2H_5$ |
| M 25 | $CH[CH_3]CH_2CH[CH_3]_2$ |
| M 26 | $CH_2$-$C[CH_3]_2C_2H_5$ |
| M 27 | $CH_2CH[CH_3]CH[CH_3]_2$ |
| M 28 | $[CH_2]_2C[CH_3]_2CH_3$ |
| M 29 | $CH[C_2H_5][CH_2]_2CH_3$ |
| M 30 | $CH_2CH[C_2H_5]C_2H_5$ |
| M 31 | $CH[CH_3]C[CH_3]_2CH_3$ |
| M 32 | $CH[CH_3]CH[CH_3]CH_2CH_3$ |
| M 33 | $C[CH_3, C_2H_5]C_2H_5$ |
| M 34 | $C[C_2H_5]CH[CH_3]_2$ |

| Verb. Nr. | A |
|---|---|
| N 1 | 2-Benzofuryl |
| N 2 | 3-Benzofuryl |
| N 3 | 4-Benzofuryl |
| N 4 | 5-Benzofuryl |
| N 5 | 6-Benzofuryl |
| N 6 | 7-Benzofuryl |

| Verb. Nr. | A |
|---|---|
| N 7 | 1-Isobenzofuryl |
| N 8 | 3-Isobenzofuryl |
| N 9 | 4-Isobenzofuryl |
| N 10 | 5-Isobenzofuryl |
| N 11 | 6-Isobenzofuryl |
| N 12 | 7-Isobenzofuryl |
| N 13 | 2-Benzo[b]thienyl |
| N 14 | 3-Benzo[b]thienyl |
| N 15 | 4-Benzo[b]thienyl |
| N 16 | 5-Benzo[b]thienyl |
| N 17 | 6-Benzo[b]thienyl |
| N 18 | 7-Benzo[b]thienyl |
| N 19 | 1-Benzo[c]thienyl |
| N 20 | 3-Benzo[c]thienyl |
| N 21 | 4-Benzo[c]thienyl |
| N 22 | 5-Benzo[c]thienyl |
| N 23 | 6-Benzo[c]thienyl |
| N 24 | 7-Benzo[c]thienyl |
| N 25 | 2-Indolyl |
| N 26 | 3-Indolyl |
| N 27 | 4-Indolyl |
| N 28 | 5-Indolyl |
| N 29 | 6-Indolyl |
| N 30 | 7-Indolyl |
| N 31 | 1-Isoindolyl |
| N 32 | 3-Isoindolyl |
| N 33 | 4-Isoindolyl |
| N 34 | 5-Isoindolyl |
| N 35 | 6-Isoindolyl |
| N 36 | 7-Isoindolyl |
| N 37 | 3-Benz[c]isoxazolyl |
| N 38 | 4-Benz[c]isoxazolyl |
| N 39 | 5-Benz[c]isoxazolyl |
| N 40 | 6-Benz[c]isoxazolyl |
| N 41 | 7-Benz[c]isoxazolyl |
| N 42 | 3-Benz[b]isoxazolyl |
| N 43 | 4-Benz[b]isoxazolyl |
| N 44 | 5-Benz[b]isoxazolyl |
| N 45 | 6-Benz[b]isoxazolyl |
| N 46 | 7-Benz[b]isoxazolyl |
| N 47 | 3-Benz[c]isothiazolyl |
| N 48 | 4-Benz[c]isothiazolyl |

| Verb. Nr. | A |
|---|---|
| N 49 | 5-Benz[c]isothiazolyl |
| N 50 | 6-Benz[c]isothiazolyl |
| N 51 | 7-Benz[c]isothiazolyl |
| N 52 | 3-Benz[b]isothiazolyl |
| N 53 | 4-Benz[b]isothiazolyl |
| N 54 | 5-Benz[b]isothiazolyl |
| N 55 | 6-Benz[b]isothiazolyl |
| N 56 | 7-Benz[b]isothiazolyl |
| N 57 | 3-Benz[b]pyrazolyl |
| N 58 | 4-Benz[b]pyrazolyl |
| N 59 | 5-Benz[b]pyrazolyl |
| N 60 | 6-Benz[b]pyrazolyl |
| N 61 | 7-Benz[b]pyrazolyl |
| N 62 | 3-Benz[c]pyrazolyl |
| N 63 | 4-Benz[c]pyrazolyl |
| N 64 | 5-Benz[c]pyrazolyl |
| N 65 | 6-Benz[c]pyrazolyl |
| N 66 | 7-Benz[c]pyrazolyl |
| N 67 | 2-Benzoxazolyl |
| N 68 | 4-Benzoxazolyl |
| N 69 | 5-Benzoxazolyl |
| N 70 | 6-Benzoxazolyl |
| N 71 | 7-Benzoxazolyl |
| N 72 | 2-Benzthiazolyl |
| N 73 | 4-Benzthiazolyl |
| N 74 | 5-Benzthiazolyl |
| N 75 | 6-Benzthiazolyl |
| N 76 | 7-Benzthiazolyl |
| N 77 | 2-Benzimidazolyl |
| N 78 | 4-Benzimidazolyl |
| N 79 | 5-Benzimidazolyl |
| N 80 | 6-Benzimidazolyl |
| N 81 | 7-Benzimidazolyl |
| N 82 | 2-[2,3-Dihydro-benzofuryl] |
| N 83 | 3-[2,3-Dihydro-benzofuryl] |
| N 84 | 4-[2,3-Dihydro-benzofuryl] |
| N 85 | 5-[2,3-Dihydro-benzofuryl] |
| N 86 | 6-[2,3-Dihydro-benzofuryl] |
| N 87 | 7-[2,3-Dihydro-benzofuryl] |
| N 88 | 1-[1,3-Dihydro-isobenzofuryl] |
| N 89 | 3-[1,3-Dihydro-isobenzofuryl] |
| N 90 | 4-[1,3-Dihydro-isobenzofuryl] |

| Verb. Nr. | A |
|---|---|
| N 91 | 5-[1,3-Dihydro-isobenzofuryl] |
| N 92 | 6-[1,3-Dihydro-isobenzofuryl] |
| N 93 | 7-[1,3-Dihydro-isobenzofuryl] |
| N 94 | 2-[2,3-Dihydro-benzo[b]thienyl] |
| N 95 | 3-[2,3-Dihydro-benzo[b]thienyl] |
| N 96 | 4-[2,3-Dihydro-benzo[b]thienyl] |
| N 97 | 5-[2,3-Dihydro-benzo[b]thienyl] |
| N 98 | 6-[2,3-Dihydro-benzo[b]thienyl] |
| N 99 | 7-[2,3-Dihydro-benzo[b]thienyl] |
| N 100 | 1-[1,3-Dihydro-benzo[c]thienyl] |
| N 101 | 3-[1,3-Dihydro-benzo[c]thienyl] |
| N 102 | 4-[1,3-Dihydro-benzo[c]thienyl] |
| N 103 | 5-[1,3-Dihydro-benzo[c]thienyl] |
| N 104 | 6-[1,3-Dihydro-benzo[c]thienyl] |
| N 105 | 7-[1,3-Dihydro-benzo[c]thienyl] |
| N 106 | 2-[2,3-Dihydro-indol] |
| N 107 | 3-[2,3-Dihydro-indol] |
| N 108 | 4-[2,3-Dihydro-indol] |
| N 109 | 5-[2,3-Dihydro-indol] |
| N 110 | 6-[2,3-Dihydro-indol] |
| N 111 | 7-[2,3-Dihydro-indol] |
| N 112 | 1-[1,3-Dihydro-isoindol] |
| N 113 | 3-[1,3-Dihydro-isoindol] |
| N 114 | 4-[1,3-Dihydro-isoindol] |
| N 115 | 5-[1,3-Dihydro-isoindol] |
| N 116 | 6-[1,3-Dihydro-isoindol] |
| N 117 | 7-[1,3-Dihydro-isoindol] |
| N 118 | 2-Chromenyl |
| N 119 | 3-Chromenyl |
| N 120 | 4-Chromenyl |
| N 121 | 5-Chromenyl |
| N 122 | 6-Chromenyl |
| N 123 | 7-Chromenyl |
| N 124 | 8-Chromenyl |
| N 125 | 1-Isochromenyl |
| N 126 | 3-Isochromenyl |
| N 127 | 4-Isochromenyl |
| N 128 | 5-Isochromenyl |
| N 129 | 6-Isochromenyl |
| N 130 | 7-Isochromenyl |
| N 131 | 8-Isochromenyl |
| N 132 | 2-Chinolyl |
| N 133 | 3-Chinolyl |

| Verb. Nr. | A |
|---|---|
| N 134 | 4-Chinolyl |
| N 135 | 5-Chinolyl |
| N 136 | 6-Chinolyl |
| N 137 | 7-Chinolyl |
| N 138 | 8-Chinolyl |
| N 139 | 1-Isochinolyl |
| N 140 | 3-Isochinolyl |
| N 141 | 4-Isochinolyl |
| N 142 | 5-Isochinolyl |
| N 143 | 6-Isochinolyl |
| N 144 | 7-Isochinolyl |
| N 145 | 8-Isochinolyl |
| N 146 | 2-Chromanyl |
| N 147 | 3-Chromanyl |
| N 148 | 4-Chromanyl |
| N 149 | 5-Chromanyl |
| N 150 | 6-Chromanyl |
| N 151 | 7-Chromanyl |
| N 152 | 8-Chromanyl |
| N 153 | 1-Isochromanyl |
| N 154 | 3-Isochromanyl |
| N 155 | 4-Isochromanyl |
| N 156 | 5-Isochromanyl |
| N 157 | 6-Isochromanyl |
| N 158 | 7-Isochromanyl |
| N 159 | 8-Isochromanyl |
| N 160 | 3-Cinnolinyl |
| N 161 | 4-Cinnolinyl |
| N 162 | 5-Cinnolinyl |
| N 163 | 6-Cinnolinyl |
| N 164 | 7-Cinnolinyl |
| N 165 | 8-Cinnolinyl |
| N 166 | 1-Phthalazinyl |
| N 167 | 4-Phthalazinyl |
| N 168 | 5-Phthalazinyl |
| N 169 | 6-Phthalazinyl |
| N 170 | 7-Phthalazinyl |
| N 171 | 8-Phthalazinyl |
| N 172 | 2-Chinoxalinyl |
| N 173 | 3-Chinoxalinyl |
| N 174 | 5-Chinoxalinyl |
| N 175 | 6-Chinoxalinyl |

| Verb. Nr. | A |
|---|---|
| N 176 | 7-Chinoxalinyl |
| N 177 | 8-Chinoxalinyl |
| N 178 | 2-Chinazolin |
| N 179 | 4-Chinazolin |
| N 180 | 5-Chinazolin |
| N 181 | 6-Chinazolin |
| N 182 | 7-Chinazolin |
| N 183 | 8-Chinazolin |
| N 184 | 3-Benzo-1,2,4-triazinyl |
| N 185 | 5-Benzo-1,2,4-triazinyl |
| N 186 | 6-Benzo-1,2,4-triazinyl |
| N 187 | 7-Benzo-1,2,4-triazinyl |
| N 188 | 8-Benzo-1,2,4-triazinyl |
| N 189 | 2-Naphthyridinyl |
| N 190 | 3-Naphthyridinyl |
| N 191 | 4-Naphthyridinyl |
| N 192 | 5-Naphthyridinyl |
| N 193 | 6-Naphthyridinyl |
| N 194 | 7-Naphthyridinyl |
| N 195 | 2-Pyrido[3,4-b]pyridinyl |
| N 196 | 3-Pyrido[3,4-b]pyridinyl |
| N 197 | 4-Pyrido[3,4-b]pyridinyl |
| N 198 | 5-Pyrido[3,4-b]pyridinyl |
| N 199 | 6-Pyrido[3,4-b]pyridinyl |
| N 200 | 8-Pyrido[3,4-b]pyridinyl |
| N 201 | 2-Pyrido[3,2-b]pyridinyl |
| N 202 | 3-Pyrido[3,2-b]pyridinyl |
| N 203 | 4-Pyrido[3,2-b]pyridinyl |
| N 204 | 6-Pyrido[3,2-b]pyridinyl |
| N 205 | 7-Pyrido[3,2-b]pyridinyl |
| N 206 | 8-Pyrido[3,2-b]pyridinyl |
| N 207 | 2-Pyrido[4,3-b]pyridinyl |
| N 208 | 3-Pyrido[4,3-b]pyridinyl |
| N 209 | 4-Pyrido[4,3-b]pyridinyl |
| N 210 | 5-Pyrido[4,3-b]pyridinyl |
| N 211 | 7-Pyrido[4,3-b]pyridinyl |
| N 212 | 8-Pyrido[4,3-b]pyridinyl |
| N 213 | 2-[1,3,2-Benzoxazinyl] |
| N 214 | 4-[1,3,2-Benzoxazinyl] |
| N 215 | 5-[1,3,2-Benzoxazinyl] |
| N 216 | 6-[1,3,2-Benzoxazinyl] |
| N 217 | 7-[1,3,2-Benzoxazinyl] |
| N 218 | 8-[1,3,2-Benzoxazinyl] |

| Verb. Nr. | A |
|---|---|
| N 219 | 2-[1,4,2-Benzoxazinyl] |
| N 220 | 3-[1,4,2-Benzoxazinyl] |
| N 221 | 5-[1,4,2-Benzoxazinyl] |
| N 222 | 6-[1,4,2-Benzoxazinyl] |
| N 223 | 7-[1,4,2-Benzoxazinyl] |
| N 224 | 8-[1,4,2-Benzoxazinyl] |
| N 225 | 1-[2,3,1-Benzoxazinyl] |
| N 226 | 4-[2,3,1-Benzoxazinyl] |
| N 227 | 5-[2,3,1-Benzoxazinyl] |
| N 228 | 6-[2,3,1-Benzoxazinyl] |
| N 229 | 7-[2,3,1-Benzoxazinyl] |
| N 230 | 8-[2,3,1-Benzoxazinyl] |
| N 231 | 2-[3,1,4-Benzoxazinyl] |
| N 232 | 4-[3,1,4-Benzoxazinyl] |
| N 233 | 5-[3,1,4-Benzoxazinyl] |
| N 234 | 6-[3,1,4-Benzoxazinyl] |
| N 235 | 7-[3,1,4-Benzoxazinyl] |
| N 236 | 8-[3,1,4-Benzoxazinyl] |
| N 237 | 3-[1,2-Benzisoxazinyl] |
| N 238 | 4-[1,2-Benzisoxazinyl] |
| N 239 | 5-[1,2-Benzisoxazinyl] |
| N 240 | 6-[1,2-Benzisoxazinyl] |
| N 241 | 7-[1,2-Benzisoxazinyl] |
| N 242 | 8-[1,2-Benzisoxazinyl] |
| N 243 | 2-[1,4-Benzisoxazinyl] |
| N 244 | 3-[1,4-Benzisoxazinyl] |
| N 245 | 5-[1,4-Benzisoxazinyl] |
| N 246 | 6-[1,4-Benzisoxazinyl] |
| N 247 | 7-[1,4-Benzisoxazinyl] |
| N 248 | 8-[1,4-Benzisoxazinyl] |
| N 249 | 2-Purinyl |
| N 250 | 6-Purinyl |
| N 251 | 8-Purinyl |
| N 252 | 1-Indanyl |
| N 253 | 2-Indanyl |
| N 254 | 3-Indanyl |
| N 255 | 4-Indanyl |
| N 256 | 5-Indanyl |
| N 257 | 6-Indanyl |
| N 258 | 7-Indanyl |
| N 259 | 1-Indenyl |

| Verb. Nr. | A |
|---|---|
| N 260 | 2-Indenyl |
| N 261 | 3-Indenyl |
| N 262 | 4-Indenyl |
| N 263 | 5-Indenyl |
| N 264 | 6-Indenyl |
| N 265 | 7-Indenyl |
| N 266 | 1-Tetralinyl |
| N 267 | 2-Tetralinyl |
| N 268 | 3-Tetralinyl |
| N 269 | 4-Tetralinyl |
| N 270 | 5-Tetralinyl |
| N 271 | 6-Tetralinyl |
| N 272 | 7-Tetralinyl |
| N 273 | 8-Tetralinyl |
| N 274 | 1-Dekalinyl |
| N 275 | 2-Dekalinyl |
| N 276 | 3-Dekalinyl |
| N 277 | 4-Dekalinyl |
| N 278 | 5-Dekalinyl |
| N 279 | 6-Dekalinyl |
| N 280 | 7-Dekalinyl |
| N 281 | 8-Dekalinyl |
| N 282 | 3-Tricyclo[5.2.1.$0^{2,6}$]decanyl |
| N 283 | 4-Tricyclo[5.2.1.$0^{2,6}$]decanyl |
| N 284 | 5-Tricyclo[5.2.1.$0^{2,6}$]decanyl |
| N 285 | 7-Tricyclo[5.2.1.$0^{2,6}$]decanyl |
| N 286 | 8-Tricyclo[5.2.1.$0^{2,6}$]decanyl |
| N 287 | 9-Tricyclo[5.2.1.$0^{2,6}$]decanyl |
| N 288 | 10-Tricyclo[5.2.1.$0^{2,6}$]decanyl |
| N 289 | 3-Tricyclo[5.2.1.$0^{2,6}$]dec-$2^{6}$-enyl |
| N 290 | 4-Tricyclo[5.2.1.$0^{2,6}$]dec-$2^{6}$-enyl |
| N 291 | 5-Tricyclo[5.2.1.$0^{2,6}$]dec-$2^{6}$-enyl |
| N 292 | 7-Tricyclo[5.2.1.$0^{2,6}$]dec-$2^{6}$-enyl |
| N 293 | 8-Tricyclo[5.2.1.$0^{2,6}$]dec-$2^{6}$-enyl |
| N 294 | 9-Tricyclo[5.2.1.$0^{2,6}$]dec-$2^{6}$-enyl |
| N 295 | 10-Tricyclo[5.2.1.$0^{2,6}$]dec-$2^{6}$-enyl |
| N 296 | 1-Norbornyl |
| N 297 | 2-Norbornyl |
| N 298 | 2-Bornyl |
| N 299 | 2-Norpinyl |

| Verb. Nr. | A |
|---|---|
| N 300 | 3-Norpinyl |
| N 301 | 1-Fluorenyl |
| N 302 | 2-Fluorenyl |
| N 303 | 3-Fluorenyl |
| N 304 | 4-Fluorenyl |
| N 305 | 5-Fluorenyl |
| N 306 | 6-Fluorenyl |
| N 307 | 7-Fluorenyl |
| N 308 | 8-Fluorenyl |
| N 309 | 9-Fluorenyl |
| N 310 | 1-Naphthyl |
| N 311 | 2-Naphthyl |
| N 312 | 3-Naphthyl |
| N 313 | 4-Naphthyl |
| N 314 | 5-Naphthyl |
| N 315 | 6-Naphthyl |
| N 316 | 7-Naphthyl |
| N 317 | 8-Naphthyl |

| Verb. Nr. | R2 |
|---|---|
| O 1 | $CH_3$ |
| O 2 | $C_2H_5$ |
| O 3 | n-$C_3H_7$ |
| O 4 | i-$C_3H_7$ |
| O 5 | n-$C_4H_9$ |
| O 6 | sek.-$C_4H_9$ |
| O 7 | tert.-$C_4H_9$ |
| O 8 | $[CH_2]_2OCH_3$ |
| O 9 | $[CH_2]_2OC_2H_5$ |
| O 10 | $[CH_2]_2O$-i-$C_3H_7$ |
| O 11 | $[CH_2]_2O$-n-$C_3H_7$ |
| O 12 | $[CH_2]_2SCH_3$ |
| O 13 | $[CH_2]_2SC_2H_5$ |
| O 14 | $[CH_2]_2S$-n-$C_3H_7$ |
| O 15 | $[CH_2]_2S$-i-$C_3H_7$ |
| O 16 | $CHF_2$ |
| O 17 | $CF_2Cl$ |
| O 18 | $CF_3$ |
| O 19 | $CH_2CF_3$ |
| O 20 | $CF_2CF_2H$ |

| Verb. Nr. | $R^2$ |
|---|---|
| O 21 | $CF_2CF_3$ |
| O 22 | $[CF_2]_2CF_3$ |
| O 23 | $CH_2Cl$ |
| O 24 | $[CH_2]_2Cl$ |
| O 25 | $CH_2F$ |
| O 26 | $[CH_2]_2F$ |
| O 27 | $[CH_2]Br$ |
| O 28 | $CH_2-OCH_3$ |
| O 29 | $CH_2-O-C_2H_5$ |
| O 30 | $CH_2-O-n-C_3H_7$ |
| O 31 | $CH_2O-i-C_3H_7$ |
| O 32 | H |
| O 33 | $i-C_4H_9$ |

| Verb. Nr. | $R^3$ |
|---|---|
| P 1 | H |
| P 2 | $CH_3$ |
| P 3 | $C_2H_5$ |
| P 4 | $n-C_3H_7$ |
| P 5 | $i-C_3H_7$ |
| P 6 | $n-C_4H_9$ |
| P 7 | $i-C_4H_9$ |
| P 8 | sek.-$C_4H_9$ |
| P 9 | tert.-$C_4H_9$ |
| P 10 | $CH_2OCH_3$ |
| P 11 | $CH_2OC_2H_5$ |
| P 12 | $CH_2O-n-C_3H_7$ |
| P 13 | $CH_2O-i-C_3H_7$ |
| P 14 | $[CH_2]_2OCH_3$ |
| P 15 | $[CH_2]_2OC_2H_5$ |
| P 16 | $[CH_2]_2O-n-C_3H_7$ |
| P 17 | $[CH_2]_2O-i-C_3H_7$ |
| P 18 | $[CH_2]_2SCH_3$ |
| P 19 | $[CH_2]_2SC_2H_5$ |
| P 20 | $[CH_2]_2S-n-C_3H_7$ |
| P 21 | $[CH_2]_2S-i-C_3H_7$ |
| P 22 | $CHF_2$ |
| P 23 | $CF_2Cl$ |
| P 24 | $CF_3$ |
| P 25 | $CH_2CF_3$ |
| P 26 | $CF_2CF_2H$ |
| P 27 | $CF_2CF_3$ |

| Verb. Nr. | $R^3$ |
|---|---|
| P 28 | $[CH_2]_2CF_3$ |
| P 29 | $CH_2Cl$ |
| P 30 | $[CH_2]_2Cl$ |
| P 31 | $CH_2F$ |
| P 32 | $[CH_2]_2F$ |
| P 33 | $[CH_2]_2Br$ |

| Verb. Nr. | $R^4$ |
|---|---|
| R 1 | 1-Naphthyl |
| R 2 | 2-Naphthyl |
| R 3 | cycl.-$C_3H_5$ |
| R 4 | cycl.-$C_4H_7$ |
| R 5 | cycl.-$C_5H_9$ |
| R 6 | cycl.-$C_6H_{11}$ |
| R 7 | cycl.-$C_7H_{13}$ |
| R 8 | cycl.-$C_8H_{15}$ |
| R 9 | 2-Furyl |
| R 10 | 2-Thienyl |
| R 11 | 2-Pyrrolyl |
| R 12 | 2-N-Methylpyrrolyl |
| R 13 | 2-Pyridyl |
| R 14 | 3-Pyridyl |
| R 15 | 2-Thiolanyl |
| R 16 | 2-Perhydro-pyrrolyl |
| R 17 | 2-Perhydro-N-methylpyrrolyl |
| R 18 | 2-Tetrahydrofuryl |
| R 19 | 3-Tetrahydrofuryl |
| R 20 | 3-Furyl |
| R 21 | 3-Perhydro-pyrrolyl |
| R 22 | 3-Pyrrolyl |
| R 23 | 3-Thiolanyl |
| R 24 | 3-Thienyl |
| R 25 | 2-Pyranyl |
| R 26 | 3-Pyranyl |
| R 27 | 4-Pyranyl |
| R 28 | 2-Tetrahydropyranyl |
| R 29 | 3-Tetrahydropyranyl |
| R 30 | 4-Tetrahydropyranyl |
| R 31 | 2-Thiopyranyl |
| R 32 | 3-Thiopyranyl |
| R 33 | 4-Thiopyranyl |

| Verb. Nr. | $R^4$ |
|---|---|
| R 34 | 2-Tetrahydro-thiopyranyl |
| R 35 | 3-Tetrahydro-thiopyranyl |
| R 36 | 4-Tetrahydro-thiopyranyl |
| R 37 | 4-Pyridyl |
| R 38 | 3-Isoxazolyl |
| R 39 | 4-Isoxazolyl |
| R 40 | 5-Isoxazolyl |
| R 41 | 3-Isothiazolyl |
| R 42 | 4-Isothiazolyl |
| R 43 | 5-Isothiazolyl |
| R 44 | 2-Oxazolyl |
| R 45 | 4-Oxazolyl |
| R 46 | 5-Oxazolyl |
| R 47 | 2-Thiazolyl |
| R 48 | 4-Thiazolyl |
| R 49 | 5-Thiazolyl |
| R 50 | 2-Imidazolyl |
| R 51 | 4-Imidazolyl |
| R 52 | 5-Imidazolyl |
| R 53 | 3-Pyrazolyl |
| R 54 | 4-Pyrazolyl |
| R 55 | 2-Pyrazinyl |
| R 56 | 2-Pyrimidinyl |
| R 57 | 4-Pyrimidinyl |
| R 58 | 5-Pyrimidinyl |
| R 59 | 2-Pyridazinyl |
| R 60 | 3-Pyridazinyl |
| R 61 | 2-Dihydropyranyl |
| R 62 | 3-Dihydropyranyl |
| R 63 | 4-Dihydropyranyl |
| R 64 | 2-Dioxolanyl |
| R 65 | 3-Dioxolanyl |
| R 66 | 4-[1,2,3-Thiadiazolyl] |
| R 67 | 5-[1,2,3-Thiadiazolyl] |
| R 68 | 4-[1,2,3-Oxadiazolyl] |
| R 69 | 5-[1,2,3-Oxadiazolyl] |
| R 70 | 3-[1,2,4-Oxadiazolyl] |
| R 71 | 5-[1,2,4-Oxadiazolyl] |
| R 72 | 2-[1,3,4-Oxadiazolyl] |
| R 73 | 5-[1,3,4-Oxadiazolyl] |
| R 74 | 2-[1,3-Dioxinyl] |

| Verb. Nr. | $R^4$ |
|---|---|
| R 75 | 4-[1,3-Dioxinyl] |
| R 76 | 5-[1,3-Dioxinyl] |
| R 77 | 6-[1,3-Dioxinyl] |
| R 78 | 2-[1,3-Dioxanyl] |
| R 79 | 4-[1,3-Dioxanyl] |
| R 80 | 5-[1,3-Dioxanyl] |
| R 81 | 6-[1,3-Dioxanyl] |
| R 82 | 2-[1,4-Dioxanyl] |
| R 83 | 3-[1,4-Dioxanyl] |
| R 84 | 5-[1,4-Dioxanyl] |
| R 85 | 6-[1,4-Dioxanyl] |
| R 86 | 2-[1,3,5-Triazinyl] |
| R 87 | 4-[1,3,5-Triazinyl] |
| R 88 | 6-[1,3,5-Triazinyl] |
| R 89 | 3-[1,2,4-Triazinyl] |
| R 90 | 5-[1,2,4-Triazinyl] |
| R 91 | 6-[1,2,4-Triazinyl] |
| R 92 | 3-[1,2,4-Oxazinyl] |
| R 93 | 4-[1,2,4-Oxazinyl] |
| R 94 | 5-[1,2,4-Oxazinyl] |
| R 95 | 6-[1,2,4-Oxazinyl] |
| R 96 | 2-[1,3,2-Oxazinyl] |
| R 97 | 4-[1,3,2-Oxazinyl] |
| R 98 | 5-[1,3,2-Oxazinyl] |
| R 99 | 6-[1,3,2-Oxazinyl] |
| R 100 | 2-[1,3,6-Oxazinyl] |
| R 101 | 4-[1,3,6-Oxazinyl] |
| R 102 | 5-[1,3,6-Oxazinyl] |
| R 103 | 6-[1,3,6-Oxazinyl] |
| R 104 | 3-[1,2,6-Oxazinyl] |
| R 105 | 4-[1,2,6-Oxazinyl] |
| R 106 | 5-[1,2,6-Oxazinyl] |
| R 107 | 6-[1,2,6-Oxazinyl] |
| R 108 | 2-[1,4-Oxazinyl] |
| R 109 | 3-[1,4-Oxazinyl] |
| R 110 | 5-[1,4-Oxazinyl] |
| R 111 | 6-[1,4-Oxazinyl] |
| R 112 | $C_6H_5$ |
| R 113 | 2-$CH_3$-$C_6H_4$ |
| R 114 | 2-$C_2H_5$-$C_6H_4$ |

| Verb. Nr. | $R^4$ |
|---|---|
| R 115 | $3\text{-}CH_3\text{-}C_6H_4$ |
| R 116 | $4\text{-}CH_3\text{-}C_6H_4$ |
| R 117 | $2\text{-}Cl\text{-}C_6H_4$ |
| R 118 | $3\text{-}Cl\text{-}C_6H_4$ |
| R 119 | $4\text{-}Cl\text{-}C_6H_4$ |
| R 120 | $2\text{-}CF_3\text{-}C_6H_4$ |
| R 121 | $3\text{-}CF_3\text{-}C_6H_4$ |
| R 122 | $4\text{-}CF_3\text{-}C_6H_4$ |
| R 123 | $2\text{-}CH_3O\text{-}C_6H_4$ |
| R 124 | $3\text{-}CH_3O\text{-}C_6H_4$ |
| R 125 | $4\text{-}CH_3O\text{-}C_6H_4$ |
| R 126 | $3\text{-}CHF_2O\text{-}C_6H_4$ |
| R 127 | $4\text{-}CHF_2O\text{-}C_6H_4$ |
| R 128 | $3\text{-}CF_3O\text{-}C_6H_4$ |
| R 129 | $4\text{-}CF_3O\text{-}C_6H_4$ |
| R 130 | $3\text{-}CClF_2O\text{-}C_6H_4$ |
| R 131 | $4\text{-}CClF_2O\text{-}C_6H_4$ |
| R 132 | $3\text{-}CH_3S\text{-}C_6H_4$ |
| R 133 | $4\text{-}CH_3S\text{-}C_6H_4$ |
| R 134 | $3\text{-}CHF_2S\text{-}C_6H_4$ |
| R 135 | $4\text{-}CHF_2S\text{-}C_6H_4$ |
| R 136 | $3\text{-}CClF_2S\text{-}C_6H_4$ |
| R 137 | $4\text{-}CClF_2S\text{-}C_6H_4$ |
| R 138 | $2\text{-}NO_2\text{-}C_6H_4$ |
| R 139 | $3\text{-}NO_2\text{-}C_6H_4$ |
| R 140 | $4\text{-}NO_2\text{-}C_6H_4$ |
| R 141 | $2\text{-}CN\text{-}C_6H_4$ |
| R 142 | $3\text{-}CN\text{-}C_6H_4$ |
| R 143 | $4\text{-}CN\text{-}C_6H_4$ |
| R 144 | $2\text{-}F\text{-}C_6H_4$ |
| R 145 | $3\text{-}F\text{-}C_6H_4$ |
| R 146 | $4\text{-}F\text{-}C_6H_4$ |
| R 147 | $3,4\text{-}Cl_2\text{-}C_6H_4$ |
| R 148 | $3\text{-}Cl\text{-}4\text{-}CH_3O\text{-}C_6H_4$ |
| R 149 | $3\text{-}Cl\text{-}4\text{-}CH_3\text{-}C_6H_4$ |
| R 150 | $2,4,6\text{-}Cl_3\text{-}C_6H_4$ |

sowie die bei A genannten Heterocyclen und Bialicyclen N1 bis 317

| Verb. Nr. | $R^5$ |
|---|---|
| S 1 | H |
| S 2 | $CH_3$ |
| S 3 | $C_2H_5$ |
| S 4 | $n-C_3H_7$ |
| S 5 | $i-C_3H_7$ |
| S 6 | $n-C_4H_9$ |
| S 7 | $i-C_4H_9$ |
| S 8 | sek.-$C_4H_9$ |
| S 9 | tert.-$C_4H_9$ |
| S 10 | $n-C_5H_{11}$ |
| S 11 | $CH[CH_3]C_3H_7$ |
| S 12 | $CH_2CH[CH_3]C_2H_5$ |
| S 13 | $[CH_2]_2CH[CH_3]CH_3$ |
| S 14 | $C[CH_3]_2C_2H_5$ |
| S 15 | $CH[CH_3]CH[CH_3]CH_3$ |
| S 16 | $CH_2-C[CH_3]_2CH_3$ |
| S 17 | $CH[C_2H_5]C_2H_5$ |
| S 18 | $n-C_6H_{13}$ |
| S 19 | $CH[CH_3]C_4H_9$ |
| S 20 | $CH_2CH[CH_3]C_3H_7$ |
| S 21 | $[CH_2]_2CH[CH_3]C_2H_5$ |
| S 22 | $[CH_2]_3CH[CH_3]CH_3$ |
| S 23 | $C[CH_3]_2C_3H_7$ |
| S 24 | $CH[CH_3]CH[CH_3]C_2H_5$ |
| S 25 | $CH[CH_3]CH_2CH[CH_3]_2$ |
| S 26 | $CH_2-C[CH_3]_2C_2H_5$ |
| S 27 | $CH_2CH[CH_3]CH[CH_3]_2$ |
| S 28 | $[CH_2]_2C[CH_3]_2CH_3$ |
| S 29 | $CH[C_2H_5][CH_2]_2CH_3$ |
| S 30 | $CH_2CH[C_2H_5]C_2H_5$ |
| S 31 | $CH[CH_3]C[CH_3]_2CH_3$ |
| S 32 | $CH[CH_3]CH[CH_3]CH_2CH_3$ |
| S 33 | $C[CH_3, C_2H_5]C_2H_5$ |
| S 34 | $C[C_2H_5]CH[CH_3]_2$ |
| S 35 | $CH_3O$ |
| S 36 | $C_2H_5O$ |
| S 37 | $n-C_3H_7O$ |
| S 38 | $i-C_3H_7O$ |

| Verb. Nr. | $R^5$ |
|---|---|
| S 39 | $n-C_4H_9O$ |
| S 40 | $i-C_4H_9O$ |
| S 41 | sek.-$C_4H_9O$ |
| S 42 | tert.-$C_4H_9O$ |
| S 43 | $CH_2OCH_3$ |
| S 44 | $CH_2OC_2H_5$ |
| S 45 | $[CH_2]_2OCH_3$ |
| S 46 | $[CH_2]_2OC_2H_5$ |
| S 47 | $[CH_2]_3OCH_3$ |
| S 48 | $[CH_2]_3OC_2H_5$ |
| S 49 | $[CH_2]_3O-n-C_3H_7$ |
| S 50 | cycl.-$C_3H_5$ |
| S 51 | cycl.-$C_4H_7$ |
| S 52 | cycl.-$C_5H_9$ |
| S 53 | cycl.-$C_6H_{11}$ |

| Verb. Nr. | $R^6$ |
|---|---|
| T 1 | H |
| T 2 | $CH_3$ |
| T 3 | $C_2H_5$ |
| T 4 | $n-C_3H_7$ |
| T 5 | $i-C_3H_7$ |
| T 6 | $n-C_4H_9$ |
| T 7 | $i-C_4H_9$ |
| T 8 | sek.-$C_4H_9$ |
| T 9 | tert.-$C_4H_9$ |

Herstellungsbeispiele

1. S(+)2-Amino-4-chlor-6-[1-cyclohexyl-ethylamino]pyrimidin

17,0 g S(+)-1-Cyclohexylethylamin und 13,5 g Triethylamin werden zu einer Mischung von 20 g 2-Amino-4,6-dichlorpyrimidin in 250 ml 1-Propanol gegeben und 7 Stunden unter Rückfluß gerührt. Man engt das Reaktionsgemisch im Vakuum ein und verteilt den Rückstand zwischen Essigester und Wasser. Die Wasserphase wird nochmals mit Essigester extrahiert, anschließend wird die gesamte organische Phase einmal mit Wasser gewaschen, über Magnesiumsulfat getrocknet und im Vakuum, zuletzt bei 90 $^0C$/0,3 mbar, eingeengt. Man erhält 28,6 g der Titelverbindung von Fp. 59 - 63$^0C$, $[\alpha]_D^{20}$ = + 3,741$^0$ - ($CH_2Cl_2$) Wirkstoffbeispiel 1.01

2. 4-Chlor-6-(furan-2-yl)methylamino-2-methoxyaminopyrimidin

a) Herstellung von 4,6-Dichlor-2-methoxyamino-pyrimidin:

Eine Lösung von 100 g (0,44 mol) 4,6-Dichlor-2-methylsulfonylpyrimidin in 400 ml N, N-Dimethylformamid wird mit 40 g (0,48 mol) O-Methylhydroxylaminhydrochlorid versetzt. Man gibt 66 g (0,48 mol) Kaliumcarbonat zu und erhitzt 10 Std. auf 90$^0C$. Man läßt abkühlen, engt ein, nimmt mit 10 %iger wässriger Salzsäure und Dichlormethan auf, trennt die Phasen, trocknet und rotiert ein. Das als Nebenprodukt entstehende 4-Chlor-6-methoxyamino-2-methylsulfonylpyrimidin kann durch Chromatographie an Kieselgel (Elutionsmittel = Cyclohexan/Essigester) abgetrennt werden. Man erhält 39 g (45 %) der Titelverbindung als weiße kristalline Masse vom Schmelzpunkt: 135 $^0C$.

b) Herstellung von 4-Chlor-6-(furan-2-yl)methylamino-2-methoxyaminopyrimidin:

Eine Lösung von 1,5 g (7,7 mmol) 4,6-dichlor-2-methoxyaminopyrimidin in 10 ml n-Propanol wird

nacheinander mit 1,14 g (8,5 mmol) Furfurylaminhydrochlorid und 1,6 g (16 mmol) Triethylamin versetzt. Man erhitzt 8 Std. zum Rückfluß, engt ein, nimmt mit Wasser und Dichlormethan auf, neutralisiert mit 10 %iger Salzsäure, trennt die Phasen, extrahiert zweimal mit Dichlormethan, trocknet die vereinten Extrakte und engt ein. Als Rückstand bleiben 1,8 g (92 %) der Titelverbindung als braunes Öl.

$^1$H-NMR ($CDCl_3$, 250 MHz) $\delta$ = 3,85 (s; 3H), 4,50 (s; 2H), 6,00 (s; 1H), 6,20 - 6,35 (m; 2H), 7,35 (d; 1H), 8,20 (s, breit; 1H).

Wirkstoffbeispiel 1.02

3. 2-Amino-4-chlor-6-[3-(1-methylethyl)-isoxazol-5-yl]-methylaminopyrimidin

8,0 g (44 mmol) 2-Amino-4-chlor-6-(propin-3-yl)aminopyrimidin in 5 ml Tetrahydrofuran wird auf 0°C gekühlt und nacheinander mit 5,3 g (44 mmol) Isobutyrylhydroxamsäurechlorid, sowie 8,9 g (88 mmol) Triethylamin versetzt. Man rührt 14 Std. bei Raumtemperatur nach, engt ein, nimmt mit Dichlormethan und Wasser auf, trennt die Phasen, trocknet die organische Phase, engt ein und reinigt durch Chromatographie an Kieselgel (Elutionsmittel: Cyclohexan/Essigester). Man erhält 6,8 g (58 %) Titelverbindung als hochviskoses Öl.

$^1$H-NMR ($CDCl_3$; 250 MHz) $\delta$ = 1,25 (d, 6H), 3,05 (sept; 1H), 4,65 (d; 2H), 5,15 (s, breit; 2H), 5,45 (s; breit; 1H), 5,85 (s; 1H), 6,05 (s; 1H).

Wirkstoffbeispiel 1.27

Entsprechend diesen in den Beispielen 1 bis 3 beschriebenen Verfahren wurden die in den folgenden Tabellen 1-3 aufgelisteten Pyrimidinderivate der Formel I erhalten.

Tabelle 1: Pyrimidinderivate der Formel I

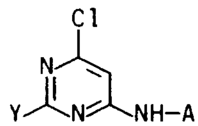

| Verb.Nr. | A | Y | Fp[°C], $^1$H-NMR in $CDCl_3$ [ppm] |
|---|---|---|---|
| 1.04 | 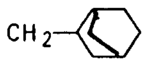 | $NH_2$ | 105 - 108 |
| 1.05 | 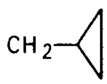 | $NH_2$ | 98 - 100 |
| 1.06 | 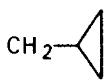 | $C_2H_5NH$ | 50 - 52 |
| 1.07 | 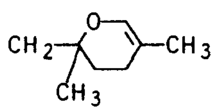 | $NH_2$ | 166 - 168 |
| 1.08 | 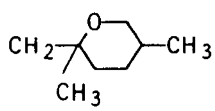 | $NH_2$ | 156 - 158 |
| 1.09 | 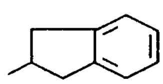 | $NH_2$ | 170 - 173 |
| 1.10 | 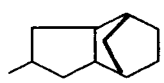 | $NH_2$ | 166 - 169 |
| 1.11 | 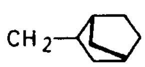 | $C_2H_5NH$ | 87 - 90 |

Tabelle 1: (Fortsetzung)

| Verb.Nr. | A | Y | Fp[°C], $^1$H-NMR in $CDCl_3$ [ppm] |
|---|---|---|---|
| 1.12 | 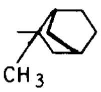 | $NH_2$ | 145 |
| 1.13 | 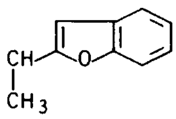 | $NH_2$ | 184 - 187 |
| 1.14 | 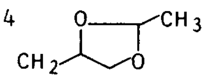 | $NH_2$ | 80 - 86 |
| 1.15 | R(-) 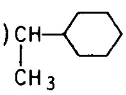 | $NH_2$ | 168<br>$[\alpha]_D^{20}$<br>-4,740° [$CH_2Cl_2$] |
| 1.16 | 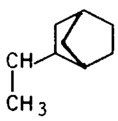 | $NH_2$ | 96 - 104 |
| 1.17 | 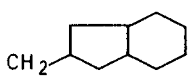 | $NH_2$ | 113 - 116 |
| 1.18 | 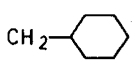 | $NH_2$ | 102 - 108 |
| 1.19 | 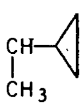 | $NH_2$ | Harz<br>$^1$H-NMR[$d_6$DMSO]<br>$CH_3$ bei 1,1 ppm d;3H |
| 1.20 | 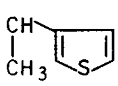 | $NH_2$ | 143 - 146 |

Tabelle 1: (Fortsetzung)

| Verb.Nr. | A | Y | Fp[°C], $^1$H-NMR in $CDCl_3$ [ppm] |
|---|---|---|---|
| 1.21 | 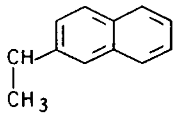 | $NH_2$ | 134 - 137 |
| 1.22 | $CH_2-C\equiv CH$ | $NHOCH_3$ | 100 - 102 |
| 1.23 | 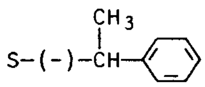 | $NHOCH_3$ | 93 - 95 |
| 1.24 | 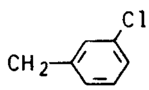 | $NHOCH_3$ | 92 |
| 1.25 | 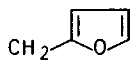 | $NHOCH_3$ | 3,85 ppm (s, 3H) $OCH_3$ |
| 1.26 | 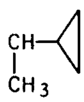 | $NHOCH_3$ | 1,15 (d, 3H) $CH_3$ |
| 1.27 | 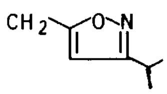 | $NH_2$ | 3,05 (s, 1H) $CH(CH_3)_2$ |
| 1.28 | 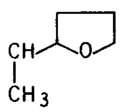 | $NH_2$ | 110 - 117 |
| 1.29 | 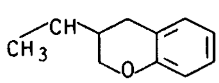 | $NH_2$ | 105 |

Tabelle 1: (Fortsetzung)

| Verb.Nr. | A | Y | Fp[°C], $^{1}$H-NMR in $CDCl_3$ [ppm] |
|---|---|---|---|
| 1.30 | 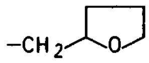 | $NH_2$ | 129 - 132 |
| 1.31 | 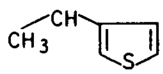 | $NHOCH_3$ | 1.6(d,3H); 3.8(s,3H); 4.8-5.1(m,1H); 5.25-5.55 (m,1H); 5.9(s,1H); 7.05 (d,1H); 7.15(d,1H); 7.35 (dd,1H); 7.5(s,br,1H) |
| 1.32 | 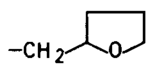 | $NHOCH_3$ | 1.8-2.15(m,4H); 3.0-4.2 (m,6H); 3.90(s,3H); 6.15(s,1H); 9.15(s,br,1H) |
| 1.33 | 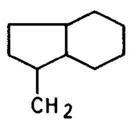 | $NHOCH_3$ | 1.10-2.10(15H); 2.9-3.35 (m,3H); 3.80(s,3H); 5.90 (s,1H); 7.9(s,br,1H) |
| 1.34 | 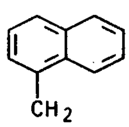 | $NHOCH_3$ | 114 - 116 |
| 1.35 | 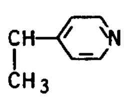 | $NH_2$ | 1.37-1.42(d/3H) 6.4(s/2H; $NH_2$) 7.65-7.7(d/1H; NH) |

Tabelle 2: Pyrimidinderivate der Formel I mit $X=OCF_3$

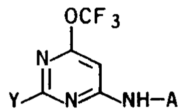

| Verb.Nr. | A | Y | Fp[°C], $^1$H-NMR in $CDCl_3$ [ppm] |
|---|---|---|---|
| 2.01 | 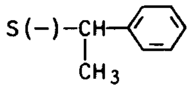 | $NH_2$ | Harz<br>5.35ppm (s/1H) NH<br>5.25 ppm (s/1H) Pyr-CH<br>4.95 ppm (s/2H) $NH_2$<br>1.55 ppm (d/3H) $CH_3$ |
| 2.02 | 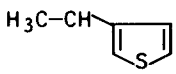 | $NH_2$ | Harz<br>5.31 (s/1H) Pyr-CH<br>5.15 (s/1H) NH<br>4.88 (s/2H) $NH_2$<br>1.50 (d/3H) $CH_3$ |
| 2.03 | 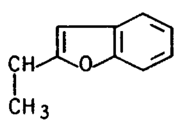 | $NH_2$ | Harz<br>6.52 (s/1H) Fur-CH<br>5.4 (s/1H) Pyr-CH<br>5.34 (s/1H) NH<br>5.05 (s/2H) $NH_2$<br>1.60 (d/3H) $CH_3$ |
| 2.04 | 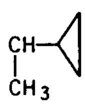 | $NH_2$ | 80 - 82 |

Tabelle 3: Pyrimidinderivate der Formel I mit X=$CF_3$

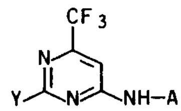

| Verb.Nr. | A | Y | Fp[°C], $^1$H-NMR in $CDCl_3$ [ppm] |
|---|---|---|---|
| 3.01 | 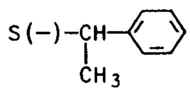 | $NH_2$ | 1.65 (d,3H); 4.95 (s,1H); 5.05-5.35(m,/1H9; 6.0 (s,1H); 7.2-7.4 (m,5H) |
| 3.02 | 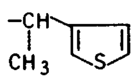 | $NH_2$ | 1.55 (d,3H); 3.55-3.7 (m,1H); 5.35 (s,br,3H); 7.05 (d,1H); 7.15 (d,1H), 7.3 (dd,1H) |
| 3.03 | 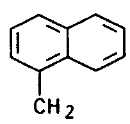 | $NH_2$ | 191 - 193 |
| 3.04 | 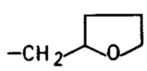 | $NH_2$ | 177 - 179 |
| 3.05 | 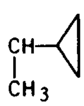 | $NH_2$ | 0.15-0.4 (m,2H); 0.45-0.6 (m,2H;) 0.8-1.0 (m, 1H); 3.25-3.55 (m, 1H); 5.0-5.6 (s,br,3H); 6.05 (s,1H); |
| 3.06 | 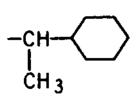 | $NH_2$ | 159 - 162 |
| 3.07 | 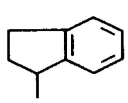 | $NH_2$ | 226 - 228 |
| 3.08 | 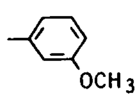 | $NH_2$ | 252 - 253 |

Anwendungsbeispiele

Die herbizide Wirkung der Pyrimidinderivate der Formel I ließ sich durch Gewächshausversuche zeigen: Als Kulturgefäße dienten Plastikblumentöpfe mit lehmigem Sand mit etwa 3,0 % Humus als Substrat. Die

Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Alopecurus myosuroides | Ackerfuchsschwanz | blackgrass |
| Avena fatua | Flughafer | wild oat |
| Abutilon theophrasti | Chinesischer Hanf | velvet leaf |
| Amaranthus retroflexus | Zurückgekrümmter | redroot pigweed |
| Cassia tora | Fuchsschwanz Gemüsekassie | sicklepod |
| Sinapis alba | Weißer Senf | white mustard |

Mit 0,75 kg/ha a.S. im Nachauflaufverfahren eingesetzt, lassen sich mit dem Beispiel 1.19 breitblättrige unerwünschte Pflanzen und Schadgräser sehr gut bekämpfen.

Aus den nachfolgenden Vergleichsversuchen geht hervor, daß die beanspruchten Pyrimidinderivate gegenüber bekannten Pyrimidinderivaten, z.B. den Vergleichssubstanzen A und B

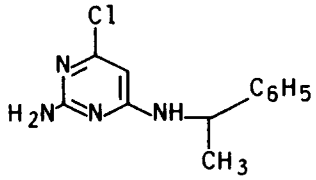

A

A bekannt aus DE-A 37 17 480, Beispiel 63

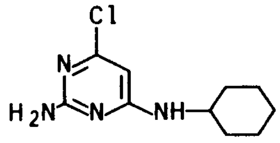

B

bekannt aus DE-A 20 65 629, Beispiel 5, sowohl eine stärkere herbizide Wirkung gegenüber Schadpflanzen als auch eine bessere Verträglichkeit gegenüber Nutzpflanzen aufweisen.

Tabelle I

Beispiele zur Bekämpfung unerwünschter breitblättriger Pflanzen und Verträglichkeit für eine Beispielkultur bei Nachauflaufapplikation von 0,375 und 0,19 kg a.S./ha im Gewächshaus

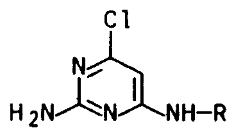 R = 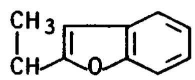 Verbdg. Nr. 1.13

R = $CH(CH_3)$-Phenyl Vergleichssubstanz A

| Testpflanzen | Schädigung in % | | | |
|---|---|---|---|---|
| | Verbindung 1.13 | | Verbindung A | |
| | 0,375 kg/ha | 0,19 kg/ha | 0,375 kg/ha | 0,19 kg/ha |
| Hordeum vulgaris (Sommergerste) | 20 | 0 | 80 | 60 |
| Amaranthus refroflexus (zurückgekrümmter Fuchsschwanz) | 100 | 100 | 100 | 100 |
| Polygonum persicaria (Flohknötrich) | 100 | 100 | 100 | 100 |
| Viola arvensis (Ackerstiefmütterchen) | 70 | 70 | 40 | 20 |

Tabelle II

Beispiele zur Bekämpfung unerwünschter breitblättriger Pflanzen und Verträglichkeit für eine Beispielkultur bei Nachauflaufapplikation von 0,375 und 0,19 kg a.S./ha im Gewächshaus

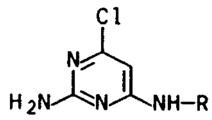

R = 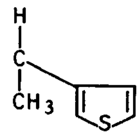 Verbdg. Nr. 1.20

R = 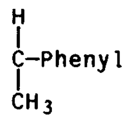 Vergleichssubstanz A

| Testpflanzen | Schädigung in % | | | |
|---|---|---|---|---|
| | Verbindung 1.20 | | Verbindung A | |
| | 0,375 kg/ha | 0,19 kg/ha | 0,375 kg/ha | 0,19 kg/ha |
| Arachis hypogaea (Erdnuß) | 20 | 10 | 40 | 40 |
| Amaranthus refroflexus (zurückgekrümmter Fuchsschwanz9 | 100 | 100 | 100 | 100 |
| Cassia tora (sicklepood) | 100 | 100 | 100 | 90 |
| Chrysanthemum (Kronenwucherblume) | 100 | 100 | 100 | 70 |

Tabelle III

Beispiele zur Bekämpfung unerwünschter breitblättriger Pflanzen bei Nachauflaufapplikation von 0,5 und 0,25 kg a.S./ha im Gewächshaus

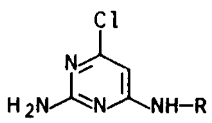

R = 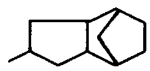 Verbdg. Nr. 1.10

R = Cyclohexyl Vergleichssubstanz B

| Testpflanzen | Schädigung in % | | | |
|---|---|---|---|---|
| | Verbindung 1.10 | | Verbindung B | |
| | 0,5 kg/ha | 0,25 kg/ha | 0,5 kg/ha | 0,25 kg/ha |
| Abuthilon theophrasti (chines. Hanf) | 100 | 100 | 0 | 0 |
| Chenopodium album (weißer Gänsefuß) | 100 | 100 | 100 | 70 |
| Ipomoea spp. (Prunkwindearten) | 100 | 100 | 50 | 20 |

## Patentansprüche

**1.** Pyrimidinderivate der Formel I

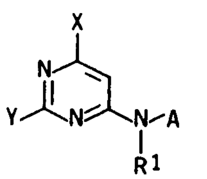

I

in welcher die Substituenten die folgende Bedeutung haben:

$R^1$ Wasserstoff oder $C_1$-$C_6$-Alkyl;

A ein bi- oder tricyclischer carbo- oder heterocyclischer Rest, der ein oder mehrere Doppelbindungen enthalten kann und unsubstituiert oder durch folgende Gruppen substituiert ist: $C_1$-$C_3$-Alkyl, $C_1$-$C_3$-Alkoxy oder Halogen; ferner $CR^2R^3R^4$, wobei

$R^2$ und $R^3$ Wasserstoff, $C_1$-$C_4$-Alkyl oder $C_1$-$C_4$-Alkyl, das ein bis drei der folgenden Gruppen trägt: $C_1$-$C_4$-Alkoxy, $C_1$-$C_4$-Alkylthio oder Halogen und

$R^4$ unsubstituiertes oder substituiertes Naphthyl mit Ausnahme von 1-Naphthyl;
unsubstituiertes oder substituiertes $C_4$-$C_8$-Cycloalkyl, ferner Cyclopropyl, falls Y $NH_2$ bedeutet und X nicht für Trifluormethyl steht;
unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff mit Ausnahme von 2-Furyl, 2-Thienyl, 2-Pyrrolyl, 2-N-Methyl-pyrrolyl, 2,3-Pyridyl, 2-Thiolanyl, 2-Perhydro-pyrrolyl und 2-Perhydro-N-methylpyrrolyl, falls $R^2$ und $R^3$ jeweils für Wasserstoff stehen und Y keine NH($C_1$-$C_4$-Alkoxy)-Gruppe bedeutet, ferner falls Y eine NH($C_1$-$C_4$-Alkoxy)-Gruppe bedeutet, unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff;
Phenyl, falls $R^2$ und/oder $R^3$ durch Halogen, Methoxy oder Methylthio substituiert sind, oder falls Y $NH_2$ und gleichzeitig X $C_1$-$C_3$-Halogenalkyl oder $C_1$-$C_3$-Halogenalkoxy bedeuten oder falls Y Mono- oder Di-$C_1$-$C_6$-alkylamino und gleichzeitig X $C_1$-$C_3$-Halogenalkoxy bedeuten, wobei der Phenylrest folgende Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro oder Cyano;

$C_2$-$C_6$-Alkyl, $C_2$-$C_6$-Alkenyl, $C_2$-$C_6$-Alkinyl, falls Y für NH($C_1$-$C_4$-Alkoxy) und gleichzeitig X für Chlor oder $C_1$-$C_3$-Halogenalkyl steht.

X Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Halogenalkylthio;

Y Halogen, $C_1$-$C_3$-Alkyl, $C_1$-$C_3$-Halogenalkyl, $C_1$-$C_3$-Alkylthio, ferner, falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch $C_1$-$C_4$-Alkoxy, $C_1$-$C_3$-Alkoxy-$C_2$-$C_3$-alkoxy, $C_1$-$C_3$-Alkylthio-$C_2$-$C_3$-alkoxy,

ferner Y für $NR^5R^6$ steht, wobei

$R^5$ Wasserstoff, $C_1$-$C_6$-Alkyl, $C_1$-$C_3$-Halogenalkyl, $C_1$-$C_4$-Alkoxy, $C_1$-$C_3$-Alkoxy-$C_1$-$C_3$-alkyl, $C_3$-$C_6$-Cycloalkyl und

$R^6$ Wasserstoff, außer wenn $R^5$ für eine Methoxygruppe und $R^4$ für Phenyl stehen, oder $C_1$-$C_4$-Alkyl bedeuten,

sowie deren Additionssalze mit organischen oder anorganischen Säuren.

**2.** Pyrimidinderivate der Formel I gemäß Anspruch 1, dadurch gekennzeichnet, daß

$R^1$ für Wasserstoff steht,

A ausgewählt ist aus der Gruppe 2-, 3-, 4-, 5-, 6-, 7-Benzofuryl, 1-, 3-, 4-, 5-, 6-, 7-Isobenzofuryl, 2-, 3-, 4-, 5-, 6-, 7-Benzo[b]thienyl, 1-, 3-, 4-, 5-, 6-, 7-Benzo-[c]thienyl, 2-, 3-, 4-, 5-, 6-, 7-Indolyl, 1-, 3-, 4-, 5-, 6-, 7-Isoindolyl, 3-, 4-, 5-, 6-, 7-Benz[c] oder -[b]isoxazolyl, 3-, 4-, 5-, 6-, 7-Benz[c] oder -[b]isothiazolyl, 3-, 4-, 5-, 6-, 7-Benz[b] oder [c]pyrazolyl, 2-, 4-, 5-, 6-, 7-Benzoxazolyl, 2-, 4-, 5-, 6-, 7-Benzthiazolyl, 2-, 4-, 5-, 6-Benzimidazolyl, 2-, 3-, 4-, 5-, 6-, 7-[2,3 Dihydro-benzofuryl], 1-, 3-, 4-, 5-, 6-, 7-[1,3-Dihydro-isobenzofuryl], 2-, 3-, 4-, 5-, 6-, 7-[2,3-Dihydro-benzo[b]thienyl], 1-, 3-, 4-, 5-, 6-, 7-[1,3-Dihydrobenzo[c]thienyl], 1-, 3-, 4-, 5-, 6-, 7-[1,3-Dihydro-benzo[c]thienyl], 1-, 3-, 4-, 5-, 6-, 7-[1,3-Dihydro-isoindol], 2-, 3-, 4-, 5-, 6-, 7-[2,3-Dihydro-indol], 2-, 3-, 4-, 5-, 6-, 7-, 8-Chromenyl, 1-, 3-, 4-, 5-, 6-, 7-, 8-Isochro-menyl, 2-, 3-, 4-, 5-, 6-, 7-, 8-Chinolyl, 1-, 3-, 4-, 5-, 6-, 7-, 8-Isochinolyl, 2-, 3-, 4-, 5-, 6-, 7-, 8-Chromanyl, 1-, 3-, 4-, 5-, 6-, 7-, 8-, Isochromanyl, 3-, 4-, 5-, 6-, 7-, 8-Cinnolinyl, 1-, 4-, 5-, 6-, 7-, 8-Phthalazinyl, 2-, 3-, 5-, 6-, 7-, 8-Chinoxa-linyl, 2-, 4-, 5-, 6-, 7-, 8-Chinazolinyl, 3-, 5-, 6-, 7-, 8-Benzo-1,2,4-triazinyl, 2-, 3-, 4-, 5-, 6-, 7-Benzopyranyl, 2-, 3-, 4-, 5-, 6-, 7-Naphthyridinyl, 2-, 3-, 4-, 6-, 7-, 8-Pyrido[3,2-b]pyridinyl, 2-, 3-, 4-, 5-, 7-, 8-Pyrido[4,3-b]pyridinyl, 2-, 3-, 4-, 5-, 6-,8-Pyrido[3,4-b]pyridinyl, 2-, 4-, 5-, 6-, 7-, 8-[1,3,2-Benzoxazinyl], 2-, 3-, 5-, 6-, 7-, 8-[1,4,2-Benzoxazinyl], 1-, 4-, 5-, 6-, 7-, 8-[2,3,1-Benzoxazinyl], 2-, 4-, 5-, 6-, 7-, 8-[3,1,4-Benzoxazinyl], 3-, 4-, 5-, 6-, 7-, 8-[1,2-Benzisoxazinyl, 2-, 3-, 5-, 6-, 7-, 8-[1,4-Benzisoxazinyl], 2-, 6-, 8-Purinyl, 1-, 2-, 3-, 4-, 5-, 6-, 7-Indanyl, 1-, 2-, 3-, 4-, 5-, 6-, 7-Indenyl, 1-, 2-, 3-, 4-, 5-, 6-, 7-, 8-Tetralinyl, 1-, 2-, 3-, 4-, 5-, 6-, 7-, 8-Deka-linyl, 3-, 4-, 5-, 7-, 8-, 9-, 10-Tricyclo-[5.2.1.$0^{2,6}$]decanyl, 3-, 4-, 5-, 7-, 8-, 9-, 10-Tricyclo[5.2.1.$0^{2,6}$]dec-$2^6$-enyl, 1-, 2-Norbornyl, 2-Bornyl, 2-, 3-Norpinyl, 1-, 2-, 3-, 4-, 5-, 6-, 7-, 8-, 9-Fluorenyl, 1-, 2-, 3-, 4-, 5-, 6-, 7-, 8-Naphthyl wobei die genannten Reste folgende Gruppen tragen können: $C_1$-$C_3$-Alkyl, $C_1$-$C_3$-Alkoxy oder Halogen;

A ferner für $CR^2R^3R^4$ steht,
wobei $R^2$ und $R^3$ Wasserstoff oder $C_1$-$C_4$-Alkyl, das ein bis drei der folgenden Gruppen tragen kann: $C_1$-$C_4$-Alkoxy- $C_1$-$C_4$-Alkylthio oder Halogen bedeuten und

$R^4$ unsubstituiertes oder substituiertes Naphthyl mit Ausnahme von 1-Naphthyl;
unsubstituiertes oder beliebig substituiertes $C_4$-$C_8$-Cycloalkyl, ferner Cyclopropyl, falls Y $NH_2$ bedeutet und X nicht für Trifluormethyl steht, ferner
unsubstituiertes oder substituiertes mono- oder bicyclisches Heterocyclyl mit ein bis drei

Heteroatomen, ausgewählt aus der Gruppe 2-Furyl, 2-Thienyl, 2-Pyrrolyl, 2-N-Methyl-pyrrolyl, 2-, 3-Pyridyl, 2-Thiolanyl, 2-Perhydro-pyrrolyl, 2-Perhydro-N-methyl-pyrrolyl, ferner 2,3-Tetrahydrofuryl, 3-Furyl, 3-Perhydro-pyrrolyl, 3-Pyrrolyl, 3-Thiolanyl, 3-Thienyl, 2-, 3-, 4-Pyranyl, 2-, 3-, 4-Dihydropyranyl, 2-, 3-, 4-Tetrahydropyranyl, 2-, 3-, 4-Thiopyranyl, 2-, 3-, 4-Tetrahydro-thiopyranyl, 4-Pyridyl, 3-, 4-, 5-Isoxazolyl, 3-, 4-, 5-Isothiazolyl, 2-, 4-, 5-Oxazolyl, 2-, 4-, 5-Thiazolyl, 2-, 4-, 5-Imidazolyl, 3-, 4-Pyrazolyl, 2-Pyrazinyl, 2-, 4-, 5-Pyrimidinyl, 2-, 3-Pyridazinyl, 2-, 3-Dioxolanyl, 4-, 5-[1,2,3-Thiadiazolyl], 4-, 5-[1,2,3-Oxadiazolyl], 3-, 5-[1,2,4-Oxadiazolyl], 2-, 5-[1,3,4-Oxadiazolyl], 2-, 4-, 5-, 6-[1,3-Dioxinyl], 2-, 4-, 5-, 6-[1,3-Dioxanyl], 2-, 3-, 5-, 6-[1,4-Dioxanyl], 2-, 4-, 6-[1,3,5-Triazinyl], 3-, 5-, 6-[1,2,4-Triazinyl], 3-, 4-, 5-, 6-[1,2,4-Oxazinyl], 2-, 4-, 5-, 6-[1,3,2-Oxazinyl], 2-, 4-, 5-, 6-[1,3,6-Oxazinyl], 3-, 4-, 5-, 6-[1,2,6-Oxazinyl], 2-, 3-, 5-, 6-[1,4-Oxazinyl], sowie die bei A genannten Heterocyclen und Alibicyclen, ausgenommen die in Anspruch 1 genannten Heterocyclen im Fall von $R^2$, $R^3$ = H und Y = NH($C_1$-$C_4$-Alkoxy), wobei die genannten Reste folgende Gruppen tragen können: $C_1$-$C_3$-Alkyl, $C_1$-$C_3$-Alkoxy, oder Halogen, ferner Phenyl, falls $R^2$ und/oder $R^3$ einen durch Halogen, Methoxy oder Methylthio substituierten Alkylrest darstellen, oder falls Y $NH_2$ und gleichzeitig X $C_1$-$C_3$-Halogenalkyl oder $C_1$-$C_3$-Halogenalkoxy bedeuten oder falls Y Mono- oder Di-$C_1$-$C_6$-alkylamino und gleichzeitig X $C_1$-$C_3$-Halogenalkoxy bedeuten, wobei der Phenylrest folgende Gruppen tragen kann: Methyl, Ethyl, Trifluormethyl, Methoxy, Ethoxy, Difluormethoxy, Trifluormethoxy, Chlordifluormethoxy, Methylthio, Difluormethylthio, Trifluormethylthio, Chlor-difluormethylthio, Halogen, Nitro oder Cyano; $R^4$ ferner $C_1$-$C_6$-Alkyl, $C_2$-$C_6$-Alkenyl, $C_2$-$C_6$-Alkinyl, falls Y für NH($C_1$-$C_4$-Alkoxy) und gleichzeitig X für Chlor oder $C_1$-$C_3$-Halogenalkyl steht;

X für Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder $C_1$-$C_3$-Halogenalkylthio steht und

Y Halogen, $C_1$-$C_3$-Alkyl, $C_1$-$C_3$-Halogenalkyl, $C_1$-$C_3$-Alkylthio, ferner falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch $C_1$-$C_4$-Alkoxy, $C_1$-$C_3$-Alkoxy-$C_2$-$C_3$-alkoxy, $C_1$-$C_3$-Alkylthio-$C_2$-$C_3$-alkoxy bedeutet

ferner Y für $NR^5R^6$ steht, wobei

$R^5$ Wasserstoff, $C_1$-$C_6$-Alkyl, $C_1$-$C_3$-Halogenalkyl, $C_1$-$C_4$-Alkoxy, $C_1$-$C_3$-Alkoxy-$C_1$-$C_3$-alkyl, $C_3$-$C_6$-Cycloalkyl und

$R^6$ Wasserstoff, außer wenn $R^5$ für eine Methoxygruppe und $R^4$ für Phenyl stehen, oder $C_1$-$C_4$-Alkyl bedeuten;

sowie deren Additionsalze mit organischen oder anorganischen Säuren.

**3.** Pyrimidinderivate der Formel I gemäß Anspruch 1, in der die Substituenten folgende Bedeutung haben:

$R^1$ Wasserstoff,;

X Chlor;

Y $NH_2$;

A eine Gruppe $CHR^3R^4$;

$R^3$ Wasserstoff, $C_1$-$C_4$-Alkyl;

$R^4$ 2-Naphthyl, $C_3$-$C_8$-Cycloalkyl, Bicyclo[2.2.1]hept-2-yl, 2-Benzofuryl, 3-Thienyl, 2-Tetrahydrofuryl, 2-Indanyl, 2-Tetrahydroindanyl, 3-Benzopyranyl.

**4.** Pyrimidinderivate der Formel I gemäß Anspruch 1, in der die Substituenten folgende Bedeutung haben:

$R^1$ Wasserstoff,;

X Chlor;

Y NH($C_1$-$C_4$)-Alkoxy;

A eine Gruppe $CHR^3R^4$;

$R^3$ Wasserstoff, $C_1$-$C_4$-Alkyl;

$R^4$ 1-Naphthyl, $C_3$-$C_8$-Cycloalkyl, Phenyl, 3-Chlorphenyl, 2-Furyl, 3-(1-Methylethyl)isoxazol-5-yl, 3-Thienyl, 3-Tetrahydrofuryl, 2-Tetrahydroindanyl.

**5.** Pyrimidinderivate der Formel I gemäß Anspruch 1, in der die Substituenten folgende Bedeutung haben:

$R^1$ Wasserstoff,;

X Trifluormethoxy;

Y $NH_2$;

A eine Gruppe $CHR^3, R^4$;
$R^3$ Wasserstoff, $C_1$-$C_4$-Alkyl;
$R^4$ 1-Naphthyl, $C_3$-$C_8$-Cycloalkyl, Phenyl, 3-Thienyl, 2-Benzofuranyl.

**6.** Pyrimidinderivate der Formel I gemäß Anspruch 1, in der die Substituenten folgende Bedeutung haben:
$R^1$ Wasserstoff,;
X Trifluormethyl;
Y $NH_2$;
A eine Gruppe $CHR^3, R^4$;
$R^3$ Wasserstoff, $C_1$-$C_4$-Alkyl;
$R^4$ 1-Naphthyl, $C_3$-$C_8$-Cycloalkyl, Phenyl, 3-Thienyl, 2-Tetrahydrofuryl, 1-Indanyl.

**7.** Herbizides Mittel, enthaltend neben inerten Zusatzstoffen mindestens ein Pyrimidinderivat der Formel I, in der die Substituenten die in Anspruch 1 gegebene Bedeutung haben.

**8.** Verfahren zur Herstellung von Verbindungen der Formel Ia

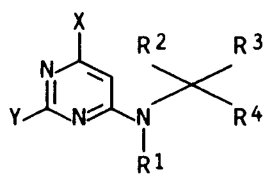

Ia ,

in der X, Y, $R^1$, $R^2$ und $R^3$ die unter Anspruch 2 genannte Bedeutung haben und $R^4$ für einen fünfgliedrigen Heteroaromaten wie unter Anspruch 2 genannt steht, dadurch gekennzeichnet, daß man den heteroaromatischen Substituenten $R^4$ im Sinne einer 1,3-dipolaren Cycloaddition an eine Verbindung der Formel Ia, in der $R^4$ für einen $C_2$-$C_6$-Alkinylrest steht, aufbaut.

**9.** Verfahren zur Bekämpfung unerwünschten Pflanzenwuchses , dadurch gekennzeichnet, daß man die unerwünschten Pflanzen und/oder die von unerwünschten Pflanzenwuchs freizuhaltende Fläche mit einer herbizid wirksamen Menge eines Pyrimidinderivats der Formel I gemäß Anspruch 1 behandelt.

**Patentansprüche für folgenden Vertragsstaat : ES**

**1.** Herbizides Mittel, enthaltend neben inerten Zusatzstoffen mindestens ein Pyrimidinderivat der Formel I

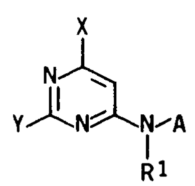

I

in welcher die Substituenten die folgende Bedeutung haben:
$R^1$ Wasserstoff oder $C_1$-$C_6$-Alkyl;
A ein bi- oder tricyclischer carbo- oder heterocyclischer Rest, der ein oder mehrere Doppelbindungen enthalten kann und unsubstituiert oder durch folgende Gruppen substituiert ist: $C_1$-$C_3$-Alkyl, $C_1$-$C_3$-Alkoxy oder Halogen; ferner $CR^2R^3R^4$, wobei
$R^2$ und $R^3$ Wasserstoff, $C_1$-$C_4$-Alkyl oder $C_1$-$C_4$-Alkyl, das ein bis drei der folgenden Gruppen trägt: $C_1$-$C_4$-Alkoxy, $C_1$-$C_4$-Alkylthio oder Halogen und
$R^4$ unsubstituiertes oder substituiertes Naphthyl mit Ausnahme von 1-Naphthyl;
unsubstituiertes oder substituiertes $C_4$-$C_8$-Cycloalkyl, ferner Cyclopropyl, falls Y $NH_2$ bedeutet und X nicht für Trifluormethyl steht;
unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff mit Ausnahme von 2-Furyl, 2-Thienyl, 2-Pyrrolyl, 2-N-Methyl-pyrrolyl, 2,3-Pyridyl, 2-Thiolanyl, 2-Perhydro-pyrrolyl und 2-Perhydro-N-methylpyrrolyl, falls $R^2$ und $R^3$ jeweils für Wasserstoff stehen und Y keine NH($C_1$-$C_4$-Alkoxy)-Gruppe bedeutet, ferner falls Y eine NH($C_1$-$C_4$-Alkoxy)-Gruppe bedeutet, unsubstituiertes oder substituiertes Bicycloalkyl oder mono- oder bicyclisches Heterocyclyl mit 1 bis 3 Heteroatomen, ausgewählt aus der Gruppe Sauerstoff, Schwefel und Stickstoff;
Phenyl, falls $R^2$ und/oder $R^3$ durch Halogen, Methoxy oder Methylthio substituiert sind, oder falls Y $NH_2$ und gleichzeitig X $C_1$-$C_3$-Halogenalkyl oder $C_1$-$C_3$-Halogenalkoxy bedeuten oder falls Y Mono- oder Di-$C_1$-$C_6$-alkylamino und gleichzeitig X $C_1$-$C_3$-Halogenalkoxy bedeuten, wobei der Phenylrest folgende Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro oder Cyano;

$C_2$-$C_6$-Alkyl, $C_2$-$C_6$-Alkenyl, $C_2$-$C_6$-Alkinyl, falls Y für NH($C_1$-$C_4$-Alkoxy) und gleichzeitig X für Chlor oder $C_1$-$C_3$-Halogenalkyl steht.

X Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, $C_1$-$C_3$-Halogenalkylthio;

Y Halogen, $C_1$-$C_3$-Alkyl, $C_1$-$C_3$-Halogenalkyl, $C_1$-$C_3$-Alkylthio, ferner, falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch $C_1$-$C_4$-Alkoxy, $C_1$-$C_3$-Alkoxy-$C_2$-$C_3$-alkoxy, $C_1$-$C_3$-Alkylthio-$C_2$-$C_3$-alkoxy,

ferner Y für $NR^5R^6$ steht, wobei

$R^5$ Wasserstoff, $C_1$-$C_6$-Alkyl, $C_1$-$C_3$-Halogenalkyl, $C_1$-$C_4$-Alkoxy, $C_1$-$C_3$-Alkoxy-$C_1$-$C_3$-alkyl, $C_3$-$C_6$-Cycloalkyl und

$R^6$ Wasserstoff, außer wenn $R^5$ für eine Methoxygruppe und $R^4$ für Phenyl stehen, oder $C_1$-$C_4$-Alkyl bedeuten,

sowie deren Additionssalze mit organischen oder anorganischen Säuren.

**2.** Herbizides Mittel gemäß Anspruch 1, enthaltend Pyrimidinderivate der Formel I, in der

$R^1$ für Wasserstoff steht,

A ausgewählt ist aus der Gruppe 2-, 3-, 4-, 5-, 6-, 7-Benzofuryl, 1-, 3-, 4-, 5-, 6-, 7-Isobenzofuryl, 2-, 3-, 4-, 5-, 6-, 7-Benzo[b]thienyl, 1-, 3-, 4-, 5-, 6-, 7-Benzo-[c]thienyl, 2-, 3-, 4-, 5-, 6-, 7-Indolyl, 1-, 3-, 4-, 5-, 6-, 7-Isoindolyl, 3-, 4-, 5-, 6-, 7-Benz[c] oder -[b]isoxazolyl, 3-, 4-, 5-, 6-, 7-Benz[c] oder -[b]isothiazolyl, 3-, 4-, 5-, 6-, 7-Benz[b] oder [c]pyrazolyl, 2-, 4-, 5-, 6-, 7-Benzoxazolyl, 2-, 4-, 5-, 6-, 7-Benzthiazolyl, 2-, 4-, 5-, 6-Benzimidazolyl, 2-, 3-, 4-, 5-, 6-, 7-[2,3 Dihydro-benzofuryl], 1-, 3-, 4-, 5-, 6-, 7-[1,3-Dihydro-isobenzofuryl], 2-, 3-, 4-, 5-, 6-, 7-[2,3-Dihydro-benzo[b]thienyl], 1-, 3-, 4-, 5-, 6-, 7-[1,3-Dihydrobenzo[c]thienyl], 1-, 3-, 4-, 5-, 6-, 7-[1,3-Dihydro-benzo[c]thienyl], 1-, 3-, 4-, 5-, 6-, 7-[1,3-Dihydro-isoindol], 2-, 3-, 4-, 5-, 6-, 7-[2,3-Dihydro-indol], 2-, 3-, 4-, 5-, 6-, 7-, 8-Chromenyl, 1-, 3-, 4-, 5-, 6-, 7-, 8-Isochro-menyl, 2-, 3-, 4-, 5-, 6-, 7-, 8-Chinolyl, 1-, 3-, 4-, 5-, 6-, 7-, 8-Isochinolyl, 2-, 3-, 4-, 5-, 6-, 7-, 8-Chromanyl, 1-, 3-, 4-, 5-, 6-, 7-, 8-, Isochromanyl, 3-, 4-, 5-, 6-, 7-, 8-Cinnolinyl, 1-, 4-, 5-, 6-, 7-, 8-Phthalazinyl, 2-, 3-, 5-, 6-, 7-, 8-Chinoxa-linyl, 2-, 4-, 5-, 6-, 7-, 8-Chinazolinyl, 3-, 5-, 6-, 7-, 8-Benzo-1,2,4-triazinyl, 2-, 3-, 4-, 5-, 6-, 7-Benzopyranyl, 2-, 3-, 4-, 5-, 6-, 7-Naphthyridinyl, 2-, 3-, 4-, 6-, 7-, 8-Pyrido[3,2-b]pyridinyl, 2-, 3-, 4-, 5-, 7-, 8-Pyrido[4,3-b]pyridinyl, 2-, 3-, 4-, 5-, 6-,8-Pyrido[3,4-b]pyridinyl, 2-, 4-, 5-, 6-, 7-, 8-[1,3,2-Benzoxazinyl], 2-, 3-, 5-, 6-, 7-, 8-[1,4,2-Benzoxazinyl], 1-, 4-, 5-, 6-, 7-, 8-[2,3,1-Benzoxazinyl], 2-, 4-, 5-, 6-, 7-, 8-[3,1,4-Benzoxazinyl], 3-, 4-, 5-, 6-, 7-, 8-[1,2-Benzisoxazinyl, 2-, 3-, 5-, 6-, 7-, 8-[1,4-Benzisoxazinyl], 2-, 6-, 8-Purinyl, 1-, 2-, 3-, 4-, 5-, 6-, 7-Indanyl, 1-, 2-, 3-, 4-, 5-, 6-, 7-Indenyl, 1-, 2-, 3-, 4-, 5-, 6-, 7-, 8-Tetralinyl, 1-, 2-, 3-, 4-, 5-, 6-, 7-, 8-Deka-linyl, 3-, 4-, 5-, 7-, 8-, 9-, 10-Tricyclo-[5.2.1.$0^{2,6}$]decanyl, 3-, 4-, 5-, 7-, 8-, 9-, 10-Tricyclo[5.2.1.$0^{2,6}$]dec-$2^6$-enyl, 1-, 2-Norbornyl, 2-Bornyl, 2-, 3-Norpinyl, 1-, 2-, 3-, 4-, 5-, 6-, 7-, 8-, 9-Fluorenyl, 1-, 2-, 3-, 4-, 5-, 6-, 7-, 8-Naphthyl wobei die genannten Reste folgende Gruppen tragen können: $C_1$-$C_3$-Alkyl, $C_1$-$C_3$-Alkoxy oder Halogen;

A ferner für $CR^2R^3R^4$ steht,
wobei $R^2$ und $R^3$ Wasserstoff oder $C_1$-$C_4$-Alkyl, das ein bis drei der folgenden Gruppen tragen kann: $C_1$-$C_4$-Alkoxy- $C_1$-$C_4$-Alkylthio oder Halogen bedeuten und

$R^4$ unsubstituiertes oder substituiertes Naphthyl mit Ausnahme von 1-Naphthyl;

unsubstituiertes oder beliebig substituiertes $C_4$-$C_8$-Cycloalkyl, ferner Cyclopropyl, falls Y $NH_2$ bedeutet und X nicht für Trifluormethyl steht, ferner
unsubstituiertes oder substituiertes mono- oder bicyclisches Heterocyclyl mit ein bis drei Heteroatomen, ausgewählt aus der Gruppe 2-Furyl, 2-Thienyl, 2-Pyrrolyl, 2-N-Methyl-pyrrolyl, 2-, 3-Pyridyl, 2-Thiolanyl, 2-Perhydro-pyrrolyl, 2-Perhydro-N-methyl-pyrrolyl, ferner 2,3-Tetrahydrofuryl, 3-Furyl, 3-Perhydro-pyrrolyl, 3-Pyrrolyl, 3-Thiolanyl, 3-Thienyl, 2-, 3-, 4-Pyranyl, 2-, 3-, 4-Dihydropyranyl, 2-, 3-, 4-Tetrahydropyranyl, 2-, 3-, 4-Thiopyranyl, 2-, 3-, 4-Tetrahydro-thiopyranyl, 4-Pyridyl, 3-, 4-, 5-Isoxazolyl, 3-, 4-, 5-Isothiazolyl, 2-, 4-, 5-Oxazolyl, 2-, 4-, 5-Thiazolyl, 2-, 4-, 5-Imidazolyl, 3-, 4-Pyrazolyl, 2-Pyrazinyl, 2-, 4-, 5-Pyrimidinyl, 2-, 3-Pyridazinyl, 2-, 3-Dioxolanyl, 4-, 5-[1,2,3-Thiadiazolyl], 4-, 5-[1,2,3-Oxadiazolyl], 3-, 5-[1,2,4-Oxadiazolyl], 2-, 5-[1,3,4-Oxadiazolyl], 2-, 4-, 5-, 6-[1,3-Dioxinyl], 2-, 4-, 5-, 6-[1,3-Dioxanyl], 2-, 3-, 5-, 6-[1,4-Dioxanyl], 2-, 4-, 6-[1,3,5-Triazinyl], 3-, 5-, 6-[1,2,4-Triazinyl], 3-, 4-, 5-, 6-[1,2,4-Oxazinyl], 2-, 4-, 5-, 6-[1,3,2-Oxazinyl], 2-, 4-, 5-, 6-[1,3,6-Oxazinyl], 3-, 4-, 5-, 6-[1,2,6-Oxazinyl], 2-, 3-, 5-, 6-[1,4-Oxazinyl], sowie die bei A genannten Heterocyclen und Alibicyclen, ausgenommen die in Anspruch 1 genannten Heterocyclen im Fall von $R^2$, $R^3$ = H und Y = NH($C_1$-$C_4$-Alkoxy), wobei die genannten Reste folgende Gruppen tragen können: $C_1$-$C_3$-Alkyl, $C_1$-$C_3$-Alkoxy, oder Halogen, ferner Phenyl, falls $R^2$ und/oder $R^3$ einen durch Halogen, Methoxy oder Methylthio substituierten Alkylrest darstellen, oder falls Y $NH_2$ und gleichzeitig X $C_1$-$C_3$-Halogenalkyl oder $C_1$-$C_3$-Halogenalkoxy bedeuten oder falls Y Mono- oder Di-$C_1$-$C_6$-alkylamino und gleichzeitig X $C_1$-$C_3$-Halogenalkoxy bedeuten, wobei der Phenylrest folgende Gruppen tragen kann: Methyl, Ethyl, Trifluormethyl, Methoxy, Ethoxy, Difluormethoxy, Trifluormethoxy, Chlordifluormethoxy, Methylthio, Difluormethylthio, Trifluormethylthio, Chlor-difluormethylthio, Halogen, Nitro oder Cyano;
$R^4$ ferner $C_1$-$C_6$-Alkyl, $C_2$-$C_6$-Alkenyl, $C_2$-$C_6$-Alkinyl, falls Y für NH($C_1$-$C_4$-Alkoxy) und gleichzeitig X für Chlor oder $C_1$-$C_3$-Halogenalkyl steht;

X für Halogen, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder $C_1$-$C_3$-Halogenalkylthio steht und

Y Halogen, $C_1$-$C_3$-Alkyl, $C_1$-$C_3$-Halogenalkyl, $C_1$-$C_3$-Alkylthio, ferner falls A einen Thienylethyl-, Furylethylrest oder einen bicyclischen Rest mit Ausnahme von Naphthyl, Chinolyl und Isochinolyl oder $R^2$ Trifluormethyl, $R^3$ Wasserstoff und $R^4$ einen wie vorgenannt substituierten Phenylrest bedeuten, auch $C_1$-$C_4$-Alkoxy, $C_1$-$C_3$-Alkoxy-$C_2$-$C_3$-alkoxy, $C_1$-$C_3$-Alkylthio-$C_2$-$C_3$-alkoxy bedeutet

ferner Y für $NR^5R^6$ steht, wobei

$R^5$ Wasserstoff, $C_1$-$C_6$-Alkyl, $C_1$-$C_3$-Halogenalkyl, $C_1$-$C_4$-Alkoxy, $C_1$-$C_3$-Alkoxy-$C_1$-$C_3$-alkyl, $C_3$-$C_6$-Cycloalkyl und

$R^6$ Wasserstoff, außer wenn $R^5$ für eine Methoxygruppe und $R^4$ für Phenyl stehen, oder $C_1$-$C_4$-Alkyl bedeuten;

sowie deren Additionsalze mit organischen oder anorganischen Säuren.

**3.** Herbizides Mittel gemäß Anspruch 1, enthaltend Pyrimidinderivate der Formel I, in der die Substituenten folgende Bedeutung haben:

$R^1$ Wasserstoff,;
X Chlor;
Y $NH_2$;
A eine Gruppe $CHR^3R^4$;
$R^3$ Wasserstoff, $C_1$-$C_4$-Alkyl;
$R^4$ 2-Naphthyl, $C_3$-$C_8$-Cycloalkyl, Bicyclo[2.2.1]hept-2-yl, 2-Benzofuryl, 3-Thienyl, 2-Tetrahydrofuryl, 2-Indanyl, 2-Tetrahydroindanyl, 3-Benzopyranyl.

**4.** Herbizides Mittel gemäß Anspruch 1, enthaltend Pyrimidinderivate der Formel I, in der die Substituenten folgende Bedeutung haben:

$R^1$ Wasserstoff,;
X Chlor;
Y NH($C_1$-$C_4$)-Alkoxy;
A eine Gruppe $CHR^3R^4$;
$R^3$ Wasserstoff, $C_1$-$C_4$-Alkyl;
$R^4$ 1-Naphthyl, $C_3$-$C_8$-Cycloalkyl, Phenyl, 3-Chlorphenyl, 2-Furyl, 3-(1-Methylethyl)isoxazol-5-yl,

3-Thienyl, 3-Tetrahydrofuryl, 2-Tetrahydroindanyl.

**5.** Herbizides Mittel gemäß Anspruch 1, enthaltend Pyrimidinderivate der Formel I, in der die Substituenten folgende Bedeutung haben:
$R^1$ Wasserstoff,;
X Trifluormethoxy;
Y $NH_2$;
A eine Gruppe $CHR^3$, $R^4$;
$R^3$ Wasserstoff, $C_1$-$C_4$-Alkyl;
$R^4$ 1-Naphthyl, $C_3$-$C_8$-Cycloalkyl, Phenyl, 3-Thienyl, 2-Benzofuranyl.

**6.** Herbizides Mittel gemäß Anspruch 1, enthaltend Pyrimidinderivate der Formel I, in der die Substituenten folgende Bedeutung haben:
$R^1$ Wasserstoff,;
X Trifluormethyl;
Y $NH_2$;
A eine Gruppe $CHR^3$, $R^4$;
$R^3$ Wasserstoff, $C_1$-$C_4$-Alkyl;
$R^4$ 1-Naphthyl, $C_3$-$C_8$-Cycloalkyl, Phenyl, 3-Thienyl, 2-Tetrahydrofuryl, 1-Indanyl.

**7.** Verfahren zur Herstellung von Verbindungen der Formel Ia

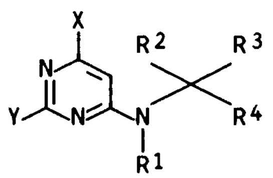

Ia ,

in der X, Y, $R^1$, $R^2$ und $R^3$ die unter Anspruch 2 genannte Bedeutung haben und $R^4$ für einen fünfgliedrigen Heteroaromaten wie unter Anspruch 2 genannt steht, dadurch gekennzeichnet, daß man den heteroaromatischen Substituenten $R^4$ im Sinne einer 1,3-dipolaren Cycloaddition an eine Verbindung der Formel Ia, in der $R^4$ für einen $C_2$-$C_6$-Alkinylrest steht, aufbaut.

**8.** Verfahren zur Bekämpfung unerwünschten Pflanzenwuchses , dadurch gekennzeichnet, daß man die unerwünschten Pflanzen und/oder die von unerwünschten Pflanzenwuchs freizuhaltende Fläche mit einer herbizid wirksamen Menge eines Pyrimidinderivats der Formel I gemäß Anspruch 1 behandelt.

Europäisches Patentamt

# EUROPÄISCHER RECHERCHENBERICHT

Nummer der Anmeldung

EP 92 11 1597

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | DE-A-3 717 480 (SHELL AGRAR)<br>* das ganze Dokument *<br>--- | 1,8,9,7 | C07D239/48<br>C07D405/12<br>C07D413/12<br>C07D401/12<br>A01N43/54 |
| D,X | PATENT ABSTRACTS OF JAPAN<br>vol. 13, no. 595 (C-672)(3943) 27. Dezember 1989<br>& JP-A-01 250 363 ( KUMIAI ) 5. Oktober 1989<br>* Zusammenfassung *<br>--- | 1,8,9,7 | |
| D,X | CHEMICAL ABSTRACTS, vol. 111, no. 28, 1989, Columbus, Ohio, US;<br>abstract no. 7430j, 'PREPARATION OF 4-(BENZYLAMINO)PYRIMIDINES AS HERBICIDES'<br>Seite 715 ;Spalte 1 ;<br>* Zusammenfassung *<br>& JP-A-63 313 777 (IDEMITSU KOSAN) 21. Dezember 1988<br>--- | 1,7,8,9 | |
| A | EP-A-0 116 961 (CELAMERCK)<br>* Ansprüche; Tabellen 1,2 *<br>--- | 1,7,8,9 | **RECHERCHIERTE SACHGEBIETE (Int. Cl.5)** |
| P,X | CHEMICAL ABSTRACTS, vol. 116, 1992, Columbus, Ohio, US;<br>abstract no. 83694s, 'PREPARATION OF 2,6-DIAMINOPYRIMIDINE DERIVATIVES AS HERBICIDES'<br>Seite 83693 ;Spalte 1 ;<br>* Zusammenfassung * | 1,2,7-9 | C07D |
| P,X | & JP-A-03 227 978 (IDEMITSU KOSAN) 8. Oktober 1991<br>--- | 1,2,7-9 | |
| P,A | EP-A-0 470 600 (CIBA-GEIGY) 12. Februar 1992<br>* Seite 44 - Seite 45; Ansprüche; Tabelle 7 *<br>----- | 1,2,6-9 | |

Der vorliegende Recherchenbericht wurde für alle Patentansprüche erstellt

| Recherchenort | Abschlußdatum der Recherche | Prüfer |
|---|---|---|
| DEN HAAG | 09 OKTOBER 1992 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)